# EUROPEAN PATENT SPECIFICATION

(11) **EP 1 513 443 B1**
(45) Date of publication and mention of the grant of the patent: **03.10.2012**
(21) Application number: 03761126.6
(22) Date of filing: 19.06.2003
(51) Int. Cl.: A61B 5/00

(54) **PERFUSION MONITOR AND SYSTEM, INCLUDING SPECIFICALLY CONFIGURED OXIMETER PROBES AND COVERS FOR OXIMETER PROBES**
PERFUSIONSMONITOR UND SYSTEM, MIT SPEZIELL KONFIGURIERTEN SONDEN UND ABDECKUNGEN FÜR OXIMETER-SONDEN
MONITEUR ET SYSTEME DE PERFUSION NON INVASIVE, SONDES D'OXYMETRE A CONFIGURATION SPECIALE ET CAPUCHONS DE SONDE

(30) Priority: 20.06.2002 US 176310
(43) Date of publication of application: 16.03.2005
(60) Divisional application: 12165265.5
(73) Proprietor: University of Florida, Gainesville, FL 32611 (US)
(72) Inventor: MELKER, Richard, Gainesville, FL 32610-0254 (US); LAYON, Joseph, A., Gainesville, FL 32610-0254 (US); WORLEY, George, Rowlett, TX 75088 (US); NAPPO, Robert, Gainesville, FL 32608 (US)
(74) Representative: Papa, Elisabetta
(86) International application number: PCT/US2003/019294
(87) International publication number: WO 2004/000114

(56) References cited:
- WO-A1-01/54575
- US-A- 4 928 691
- US-A- 5 275 159
- US-A- 5 309 908
- US-A- 5 311 865
- US-A- 5 335 659
- US-A- 5 413 102
- US-A- 5 507 286
- US-A- 5 743 857
- US-A- 5 788 634
- US-A- 5 800 349
- US-A- 5 810 724
- US-A- 5 817 010
- US-A- 6 112 107
- US-A- 6 144 867
- US-A1- 2002 016 537
- US-B1- 6 334 065

## Description

### Field of the Invention

The present invention relates to the field of non-invasive measurement of signals indicating arterial blood oxygen saturation by means of pulse oximetry.

### Background of the Invention

Diseases, acute injuries, and other conditions can adversely affect blood flow to and in the limbs. In a general sense, agents and factors that may affect and lower circulation to the limbs, also known as peripheral circulation, include certain drugs, especially vasoconstrictors, poor perfusion per se due to shock, such as results from low blood volume, or septic or cardiogenic shock, certain traumas, external pressure (as from bums), hypothermia, and other mechanical abnormalities or injuries. In particular, decreased peripheral circulation may be caused by a number of disorders within the body including, but not limited to, atherosclerosis, Raynaud's disease, Buerger's disease, chronic obstructive pulmonary diseases (COPD), and embolic occlusive disease.

Poor blood flow reduces the amount of oxygen that is carried in the blood stream to cells. Emergency rooms, intensive care units, burn units, operating rooms, and ambulances treat a variety of critically ill patients in need of continuous monitoring of real time hemoglobin saturation and/or blood pressure readings. If oxygen levels in the blood become very low at peripheral sites, a variety of problems may occur which include inadequate resuscitation, cell death or necrosis that can lead to non-healing lesions, gangrene and amputation of limbs. Also, in progressive diabetes and other conditions that may result in atherosclerosis that affect peripheral circulation and perfusion, non-invasive measurement of circulation and/or resistance status is useful to monitor the progression of the disease and the effectiveness of treatments.

Also, many patients, especially among the elderly, are on chronic oxygen therapy; they are in need of supplemental oxygen on a routine basis. Such patients may have impaired and/or diminished cardiopulmonary capacity. When such patients are ambulatory, their supply of oxygen (usually a tank of compressed oxygen or liquid oxygen) must be transported with them wherever they travel. Oxygen from such supply passes through a regulator and thence, typically, via a tube to the nose where it is inhaled (e.g., via a nasal cannula or the like). Alternatively, the oxygen may be delivered by a cannula directly into the trachea (transtracheal supplemental oxygen). Arrangements of the present invention combine the supply of oxygen or oxygen-rich air with a pulse oximeter that adjusts the release of the supply to better match the actual bodily requirement based on the measured blood oxygen saturation. A pulse oximeter that receives the signal from the pulse oximeter probe is located a distance from the probe itself, and provides a blood oxygen saturation measurement to the user (and/or to a remote monitor), and/or, in certain arrangements acts to adjust the inflow rate or quality of the oxygen or oxygen-rich gas being supplied. This, depending on each particular user and his/her baseline settings, can either extend the life of a given supply of compressed oxygen or oxygen-rich gas, or provide oxygen or oxygen-rich gas on a more accurate, as-needed basis, in the latter case improving the health and/or performance of the user.

As to the latter benefit of this aspect of the present arrangement provision of an accurate, as-needed supply of oxygen reduces the risk of and/or alleviates problems of hypoxia that are associated with improper adjustment of supplemental oxygen to patients in need thereof. Hypoxia, low oxygen delivery, or hypoxemia, low oxygen tension in the blood, cause a number of maladies including polycythemia (increased hematocrit) which leads to abnormal clotting. Polycythemia is a compensatory mechanism to chronic hypoxemia that typically builds up over weeks to months. It is typical in persons with chronic lung disease (and also persons living at high altitudes).

A more immediate, primary physiologic compensatory response to oxygen deficit is increased cardiac output. This is normal, such as during increased physical exertion. However, in persons who have impaired cardiocirculatory reserve, increased cardiac output in response to low arterial oxygen level can, under certain circumstances, eventually lead to death. The second immediate physiologic compensatory response to oxygen deficit is the extraction of more oxygen from hemoglobin within the capillaries of the body's organs. This normally happens either during an increase in oxygen demand (i.e., exercise, fever, shivering, etc.), or during normal demand but decreased oxygen delivery (i.e., due to inadequate blood flow, anemia, hypoxia). In such instances, metabolically active cells draw additional oxygen from the red blood cells which ultimately resulting in a decrease in the mixed venous blood's oxygen saturation falling from a typical 65% to 80% level to levels as low as 32% (*see* Hemodynamic Monitoring - Invasive and Noninvasive Clinical Application, by Gloria Oblouk Darovic, 3rd Ed., 2002, Chapter 12). Chronic hypoxemia can lead to a switch by metabolically active cells to anaerobic metabolism, which, especially in patients with limited cardiopulmonary reserve, can lead to lactic acidosis and eventually death.

Hypoxemia also causes cognitive dysfunction either acutely or chronically which can lead to early dementia and death. Generally, based on the compensatory mechanisms and effects on body tissues, chronic hypoxemia may affect all organs in the body leading to failure of any or all organs.

In general, blood oxygen levels are currently measured by pulse oximetry, which can be divided into transmittance and reflectance types. Transmittance, or transillumination oximetry, involves the process whereby a sensor measures light extinction as light passes *through* a portion of blood-perfused tissue. Light is transmitted from one side of a portion of blood-perfused tissue, and is recorded by a sensor situated across the portion of tissue. Reflectance oximetry, on the other hand, has both the light source and the sensor on one side of the tissue, and measures reflectance back from the tissue. For both types of oximetry, multiple signals from the light sensor, or detector, are used to estimate the oxygen saturation and pulse rate from changes in absorption of the light detected throughout blood pulse cycles. The technology is based on the differential absorbence of different wavelengths of light by different species of hemoglobin.

Conventional pulse oximetry measurement in certain classes of patients, for instance severely burned patients, can be a significant challenge, yet this monitoring data is vital in operating room and intensive care settings. Most current pulse oximetric approaches depend upon available peripheral sites permitting transillumination oximetry which is sufficient for most surgical conditions and procedures. However, in one example, patients with severe burns often have only a few sites suitable for the effective placement of the transmitting pulse oximeter sensor. These patients often have severe circulatory compromise rendering the current peripheral pulse oximeters less effective.

The technology of pulse oximeters is well known (See "Pulse Oximetry: Principles and Limitations," J.E. Sinex, Am. J. Emerg. Med., 1999, 17:59-66). Pulse oximetry includes a sensor, or probe, with light source(s) generating at least two different wavelengths of light, and a detector placed across a section of vascularized tissue such as on a finger, toe, or ear lobe. Pulse oximetry relies on the differential absorbance of the electromagnetic spectrum by different species of hemoglobin. In a typical system, two distinct wavelength bands, for instance 650-670 nm and 880-940 nm, are used to detect the relative concentrations of oxygenated hemoglobin (oxyhemoglobin) and non-oxygenated reduced hemoglobin, respectively. The background absorbance of tissues and venous blood absorbs, scatters and otherwise interferes with the absorbance directly attributable to the arterial blood. However, due to the enlargement of the cross-sectional area of the arterial vessels during the surge of blood from ventricular contraction, a relatively larger signal can be attributed to the absorbance of arterial hemoglobin during the systole.

By averaging multiple readings and determining the ratio peaks of specific wavelengths, a software program can estimate the relative absorbance due to the arterial blood flow. First, by calculating the differences in absorption signals over short periods of time during which the systole and diastole are detected, the peak net absorbance by oxygenated hemoglobin is established. The signals typically are in the hundreds per second. The software subtracts the major "noise" components (from non-arterial sources) from the peak signals to arrive at the relative contribution from the arterial pulse. As appropriate, an algorithm system may average readings, remove outliers, and/or increase or decrease the light intensity to obtain a result. The results from one site provide a measurement of arterial oxygen saturation at that site, and also allows calculation of the shape of the pulse at the placement site of the probe, which can be developed into a plethysymograph. Among the various sources of signal interference and modification, it is noted that the shape of red blood cells changes during passage through arterial and venous vessels. This change in shape affects scattering of the light used in pulse oximetry. Algorithms are designed to correct for such scattering.

More sophisticated pulse oximetry systems detect at more than merely two bands, such as the 650-670 nm and 880-940 nm wavelength bands. For instance, the pulse oximetry article from a uni-erlangen web site stated that four LEDs, at 630, 680, 730 and 780 nm, each with 10 nm bandwidths, can determine the four common species of hemoglobin. The article further calculated that the detection of nine wavelengths in the range of 600 to 850 nm would provide greater accuracy in assessing these four forms of hemoglobin, oxyhemoglobin (O₂Hb), reduced hemoglobin (HHb), methemoglobin (MetHb), and carboxyhemoglobin (COHb). As used in the present invention, the term "pulse oximeter" or "oximeter" is meant to include all designs and types of pulse oximeters, including current and later developed models that transmit and detect at more than two wavelengths associated with absorption differences of these hemoglobin species.

At present, peripheral vascular resistance can only be measured invasively, or non-invasively by skilled technicians using Doppler flow devices. The use of Doppler and Doppler waveform analysis is now a standard investigation technique for obtaining measurements in blood flow resistance patients with possible circulatory disorders. For example, Dougherty and Lowry (J. Med. Eng. Technol., 1992: 16:123-128) combined a reflectance oximeter and a laser Doppler flowmeter to continuously measure both blood oxygen saturation and perfusion.

A number of patents have been issued directed to monitors, sensors and probes for use in pulse oximetry procedures. For instance, U.S. Patent No. 6,334,065, issued on December 25, 2001 to Al-Ali, et al., discloses a stereo pulse oximeter that provides for simultaneous, non-invasive oxygen status and photoplethysmograph measurements at both single and multiple sites. The invention is directed to the detection of neonatal heart abnormalities, particularly related to defects of heart-associated vessels, and specifically directed to Persistent Pulmonary Hypertension in Neonates (PHHN), Patent Ductus Arteriosis (PDA), and Aortic Coarctation. All of these conditions result in a flow of differentially oxygenated blood to different peripheral extremities. For instance, in PHHN and PDA, the blood that flows to the right hand is unaffected by the abnormal shunt that results in less oxygenated blood flowing to other areas. Thus, comparison of oxygen saturation values between a pulse oximeter sensor at the right hand and at, for instance, a foot site, is stated to detect or confirm the diagnosis of such neonatal heart abnormalities. Continuous monitoring with such pulse oximetry also is proposed, to provide feedback on the effectiveness of treatments or surgery to deal with these neonatal cardio/cardiopulmonary conditions. U.S. Patent No. 6,334,065 does not address the use of two probes for detection, confirmation, or monitoring of perfusion- and resistance-related conditions in the patient. Such conditions would not be expected in a neonatal patient, and are instead more likely found in aging patients and in patients with certain accident conditions unrelated to neonatal heart and heart-associated vessel anomalies.

U.S. Patent No. 6,263,223 was issued on July 17, 2001 to Shepard et al., and teaches a method for taking reflectance oximeter readings within the nasal cavity and oral cavity and down through the posterior pharynx. Whereas the conventional transillumination pulse oximeter probe detects the light not absorbed or scattered as it crosses a vascularized tissue covered by skin (i.e., the LEDs and photodetector are separated by the tissue), a reflectance oximeter probe detects light by backscattering of light that traverses vascularized tissue not bounded by skin and is reflected back to a detector positioned on the same side of the tissue as the LEDs (e.g., on tissue in the mouth). The method includes inserting a reflectance pulse oximeter sensor into a cavity within a subject's skull and contacting a capillary bed disposed in the cavity with the reflectance pulse oximeter sensor. The method uses standard pulse oximeter sensor probes placed over capillary beds close to a buccal surface, posterior soft palate, hard palate or proximal posterior pharynx, including the tongue, nares or cheek. Reflectance pulse oximetry at these sites determines arterial oxygen saturation. One major problem with this device is that it does not permit cross-site comparisons of oxygen saturation values between several tissue sites. In addition, the pulse oximeter device used in this invention is an elongated tube that is inserted far into the nasal or oral cavity down into the pharynx, which is a highly invasive procedure.

U.S. Patent No. 4,928,691, issued on May 29, 1990 to Nicolson et al., and currently withdrawn, discloses a non-invasive, electro-optical sensor probe and a method for its use. The sensor is enabled to measure light extinction during transillumination of a portion of blood-perfused tissue and to calculate the oxygen saturation and pulse rate from changes in absorption of the light detected. The sensor probe is placed at a central site such as the tongue, cheek, gum or lip of the patient and provides continuous assessment of arterial oxygen saturation and pulse rate. The sensor is malleable and extremely flexible, and is stated to conform to the structure of the skin and underlying tissue. U.S. Patent No. 4,928,691 states that measurement at the preferred central sites provide accurate oxygen saturation and pulse readings for "patients with lowered or inconsistent peripheral perfusion." Critically, the probes according to U.S. Patent No. 4,928,691 are highly flexible, leading to a high likelihood that upon typical movement of the patient there would be mal-alignment between the light source(s) and sensor, resulting in skewed, non-usable, or unreliable signals and results. Also, there is no teaching or suggestion to compare oxygen saturation values between several tissue sites to identify, characterize, or monitor peripheral perfusion conditions in such patients.

U.S. Patent No. 5,218,962 was issued on June 15, 1993 to Mannheimer et al., teaches a pulse oximetry system which monitors oxygen saturation and pulse rates by sensing the blood characteristics at two or more peripheral sites. The device includes one or more pulse oximetry probes which passes light through unique regions of tissue and a sensor which detects the amount of light passing through the tissue, and an instrument that independently calculates oxygen saturation level within each region. The difference in values represents how much the oxygen saturation of the first region of tissue differs from the oxygen saturation of the second region of tissue. When the difference between the two values is below a set threshold, the '962 patent attributes this to a sufficiently high probability that the value is true, and displays an oxygen saturation value that is a function of the two independent values. Where there is a difference greater than a set threshold, no oxygen saturation value is displayed. Thus, the '962 patent attributes substantial differences between two sites to be due to error, rather than to an indication of a problem with peripheral perfusion and/or resistance.

WIPO Publication No. WO0021435A1, to Barnett et al., was published April 20, 2000. This publication teaches a non-invasive spectrophotometric examination and monitoring of blood metabolites in multiple tissue regions on an ongoing and instantaneous basis. The method includes attaching multiple sensors to a patient and coupling each sensor to a control and processing station enabled to analyze signals conveyed thereto. The control and processing station visually displays the data from multiple sites for direct mutual comparison of oxygen saturation values from multiple sites. A key aspect of the invention is the use of a "near" and a "far" (or "deep") detector at each detection site. Based on the positioning of the light-generating devices and the near and far sensors, the far sensor receives absorption signals from deeper inside the brain tissue. In a basic configuration, the "near" sensor, or detector, principally receives light from the source whose mean path length is primarily confined to the layers of skin, tissue, and skull, while the "far" detector which receives light sprectra that have followed a longer mean path length and traversed a substantial amount of brain tissue in addition to the bone and tissue traversed by the "near" detector. Other configurations indicate receptors receive signals from sources across the entire brain cross-section. This is stated to provide information about, by calculation differences, the condition of the deeper tissue, in particular the brain. The method is directed to compare oxygen saturation values for cerebral tissue, such as comparing the two hemispheres during surgery. The WO0021435A1 invention distinguishes itself from standard pulse oximetry of arteries close to the surface of the body, and focuses primarily on analysis of deeper tissues and organs. The application does not teach a method to measure "surface" peripheral or central tissue sites for development of information regarding perfusion status.

WIPO Publication No. WO0154575A1, to Chen et al., was published on Aug. 2, 2001. This publication teaches a non-invasive apparatus and method for monitoring the blood pressure of a subject. A monitor is used for continuous, non-invasive blood pressure monitoring. The method includes using sensors to detect a first blood pressure pulse signal at a first location on patient and detecting a second blood pressure pulse signal at a second location on the patient; measuring a time difference between corresponding points on the first and second blood pressure pulse signals; and, computing an estimated blood pressure from the time difference. The first and second sensors are placed at locations such as a finger, toe, wrist, earlobe, ankle, nose, lip, or any other part of the body where blood vessels are close to the surface of the skin of a patient where a blood pressure pulse wave can be readily detected by the sensors, and/or where a pressure pulse wave from the patient's heart takes a different amount of time to propagate to the first location than to the second location.

In one regard, a superior monitor system would be able to provide real-time continuous measurements of signals that would be analyzed to provide arterial oxygen saturation, blood pressure, and pulse rate. A superior monitor system would utilize at least two pulse oximeter probes, one of which is placed at a highly perfused central tissue, such as the lip, tongue, nares, cheek, and a second probe placed at a typically less perfused areas such as a finger or toe. Also, in some situations, a peripheral probe may be placed at sites in or distal from areas that may be or are affected by disease- or accident-related diminished blood perfusion to tissues.

An additional aspect of a superior oximeter system provides both an inflow means of oxygen or oxygen-rich gas to a patient in need thereof, and an integral or adjoining pulse oximeter probe. This aspect is in conjunction with the above-described two pulse oximeter probe system, or in a system that only has one oximeter probe. In either case, one pulse oximeter probe, positioned at the nose or mouth, detects the levels of oxygenation saturation of blood in the patient, and detection of low or lowering oxygenation saturation levels results in one or more of: setting off a local or remote alarm or message; increasing the flow of oxygen or oxygen-rich gas to said patient. Likewise, detection of higher or increasing oxygenation levels results in one or more of: setting off a local or remote alarm or message; decreasing the flow of oxygen or oxygen-rich gas to said patient. Preferably, the pulse oximeter probe at the nose or mouth is integral with the delivery means of the oxygen or oxygen-rich gas. Preferably, the control of oxygenation levels is by signaling to (manually or automatically) adjust a valving mechanism that controls output flow from a source of auxiliary of oxygen or oxygen-rich gas. By such feedback mechanism the quantity of oxygen or oxygen-rich gas is conserved, and the needs of such patient are more closely attuned to the fluctuations in oxygen demand during activities at varying levels of exertion during a period of time.

As to references that pertain to the combining of a pulse oximeter with a system to control the inflow of oxygen or other oxygen-rich gas to a patient in need thereof, the following U.S. patents, and references contained therein, are considered to reflect the state of the current art: 4,889,116; 5,315,990; 5,365,922; 5,388,575; 6,371,114; and 6,512,938. None of these references are specifically directed to a combined, preferably integral combined pulse oximeter sensor/nasal cannula, which, when combined with an oximeter, or with an oximeter that controls the inflow of such oxygen or other oxygen-rich gas to the patient. US 5413102 discloses an oximeter according to the preamble of claim 1.

### Summary of the Invention

One aspect not part of the not part present invention relates to a novel non-invasive vascular perfusion/resistance monitor system having at least two pulse oximeter probes positioned at locations on the body of a patient, the signals from which may be capable of indicating a problem with peripheral perfusion and/or resistance. In practice each probe emits at least two different light frequencies, such as by light-generating diodes (LEDs), and such emitted light is detected by at least one light detector, such as a photodiode detector. A general-purpose computer or a special purpose computer is employed to perform complex mathematical computations based, typically, on the signal intensity and timing from the at least two pulse oximeter probes, and on signals from the light detectors of each of the probes. Proper analysis by software programming in such general-purpose computer or special purpose computer outputs results to a display, printer, etc. that suggests or indicates (depending on relative differences in the signals at different locations, and upon other conditions) whether a condition of diminished or abnormal vascular perfusion/resistance may exist in a selected body area. The system also monitors changes in such conditions during treatment interventions.

Further software programming provides for a signal to a user of the device to alert the user when signals from a central or a non-central probe are of such low pulse amplitude that either the probe needs repositioning or that the patient is experiencing extremely low pulse at the probe site (and is therefore in need of acute intervention). The software program also converts the signals from the light detectors to calculate various oxygen saturation values and various blood pressure values (either simultaneously or separately). These values are used for evaluating the vascular perfusion/resistance and/or blood pressure of a patient based on the locations of the two or more probes.

Each probe is designed for monitoring blood oxygen saturation and/or blood pressure at different vascular bed sites on a patient. Critically, one of the at least two sites on a patient is at what is designated a "central source" site ("CSS"). The inventors have discovered that flow directed through the carotid artery and detected at CSS sites, such as the lip, tongue, nasal nares, and facial cheek, are typically strong and unaffected by perfusion-lowering conditions. In patients who do not have perfusion-lowering conditions, a second or third probe site at "non-central" site (NCS), such as an extremity (i.e., fingers, toes, etc.), provides oxygen saturation and pulse values fairly comparable to values from the CSS. However, when a patient has a perfusion-lowering condition, the probe site at an affected extremity provides noticeably different oxygen saturation and pulse values compared to the CSS values. The difference in saturation values between the CSS and one or more sites is then used to assess peripheral vascular resistance, perfusion and/or peripheral vascular disease.

As used in this disclosure, when a particular wavelength or band of wavelength is stated at which an LED or other light-generating source emits light, it is understood that such light-generating source may and probably does emit light across a broader range. However, what is meant by such statement is that such light-generating source is designed to emit at a frequency curve which has a peak at or near such stated wavelength or band. It is further understood that any known means of limiting non-desired light energy, such as by selective filtration, may be used in conjunction with such light-generating sources to improve the accuracy and/or precision of the emissions of such light-generating sources.

As used in this disclosure, a "pad" is meant to indicate a housing, or an enclosure, over a light-generating or a light-sensing device on the probe, which provides a barrier to fluids, and permits transmission of light of the relevant wavelengths to the present invention. A typical pad has a composition of clear plastic.

As used in this disclosure, a "conductor" is meant to indicate any physical pathway or any system that communicates a signal or electricity from a first to a second location. Signals and electricity can be conducted by conventional means, such as by sending electrical impulses along a continuous copper wire, by more sophisticated means, such as by converting the signals into radio waves and transmitting these waves such that a receiver receives the signals and thereafter sends them to the controller, or by any other way now known or later developed.

As used in this disclosure, whether or not so stated in a particular sentence, the term "oxygen" may be taken to mean "oxygen or any oxygen-rich air or other gas mixture that contains oxygen" which is used for provision of oxygen to a patient or to a person in need thereof. The context of a particular usage in this disclosure indicates whether this broader definition is to be used, or whether a particular example is referring instead to the use of pure oxygen exclusively.

While some researchers have attempted to gauge accuracy by comparing the results from two different pulse oximeter probe sites (see U.S. 5,218,962), and other researchers generally recognized that "central" sites are generally more reliable and responsive than "peripheral" sites (see U.S. 6,263,223, and 4,928,691), the present invention recognizes the reasons for the inconsistently different results between CSS and non-CSS sites. Specifically, patients having compromised peripheral circulation and/or resistance will tend to have lower peripheral values than patients without such compromised conditions. By such recognition, detection and monitoring impaired peripheral circulation is possible through the present disclosure. The monitoring system, in certain arrangements additionally provides an indication of vascular resistance through continuous monitoring of the transit time difference of the blood oxygen saturation values and the blood pressure values between the two sites.

An arrangement not-part of the present invention to provides a monitoring system which includes two pulse oximeter sensors, or probes, connected to a monitor system as a non-invasive means for continuously measuring blood oxygen saturation values and/or blood pressure and/or pulse values, wherein the system detects and monitors changes in vascular perfusion and resistance in a patient. The overall system particularly assesses differences in peripheral blood flow which may be impaired in certain illnesses and accident conditions.

An object of the present invention is to provide probes functionally constructed to provide more reliable signal reception and transmission for patients, such as those in ICU, surgery, post-operative care, and patients with respiratory, circulatory problems, or under anesthetics. In particular, pulse oximeter probes are configured to be placed, respectively, across the lip or cheek, in the nostrils of the nose, and on the tongue.

Thus, one object of the invention is to provide a novel configuration of an oximeter probe that is well-suited for placement across the lip of the mouth of a patient, or the cheek of a patient, in which one side of the probe is situated outside the oral cavity and a second side is positioned inside the mouth cavity, and the tissue between the two sides is assessed by transillumination pulse oximetry.

An arrangement not part of the invention provides a novel configuration of an oximeter probe that is well-suited for placement at the nasal cavity of a patient, in which one side of the probe is situated to the left side of the nasal septum, and a second side is positioned to the right side of the nasal septum, and the tissue between the two sides is assessed by transillumination pulse oximetry. This design, in a preferred arrangement also functions to provide oxygen to the patient through channels provided in the structure of the probe.

An arrangement not part of the invention provides a novel configuration of an oximeter probe that is well-suited for placement on both sides of either the right or the left nasal alar (i.e., the alar nari). One side of the probe is situated to the outside of the nasal nari, and a second side is positioned to the inside of the nasal nari, and the tissue between the two sides is assessed by transillumination pulse oximetry.

Another arrangement not part of the invention provides a novel configuration of an oximeter probe that is well-suited for placement on the tongue of a patient, in which one part of the probe is situated along one surface of the tongue, and an opposing part is positioned in such a manner as to capture a section of the tongue such that a transilluminable cross-section of tongue tissue is held between the two probe parts, and the tongue tissue between the two probe parts is assessed by transillumination pulse oximetry.

Another arrangement not part of the present invention provides pulse oximeter probes dimensioned and configured to be expandable, spring-loaded, and flat surfaced for utilizing measurements on extremities and earlobes; bucal mucosal- bucal surface or dorsal ventral portion of the tongue; and properly sized configurations for the nasal alars (i.e., alar nares) and cheek and/or tongue for critically ill, burned, or traumatized patients. A related object is to provide a configuration for an oximeter probe that utilizes two opposed, substantially flat probe surfaces that tend toward each other, such as by spring tensioning.

A further arrangement not part of the present invention provides a monitoring system that measures vascular resistance and/or perfusion continuously to improve volume resuscitation and/or drug therapy.

It is still a further object of the present invention to provide a monitoring system that can be used as a multi-probe pulse oximeter to monitor blood oxygen saturation differences, pulse transit time differences, or blood pressure, or any combination thereof.

Still another arrangement not part of the present invention provides specifically constructed probes used to transmit and receive the light to vascular bed sites that are not normally available for use due to burns, trauma, and surgery on the extremity.

A still another arrangement not part of the present invention provides a monitoring system that is easily fabricated from low cost material and is adaptable for use in an operating room, intensive care unit, emergency room or other areas to treat patients in need of hemodynamic monitoring.

Still another arrangement not part of the present invention provides a pulse oximeter probe and a supply of oxygen or oxygen-rich air, in combination, and functioning in concert with each other and with oximetry circuitry, such that the level and trend in blood oxygen saturation are determined by the pulse oximeter and changes in blood oxygen saturation direct a change (i.e., an increase or a decrease) in the release of oxygen or oxygen-rich air to the patient whose blood oxygen saturation is being measured. In one type of control of the flow of oxygen or oxygen-rich air, an electronic regulator is controlled by signals from a processor that receives data from the pulse oximeter.

Thus, one particular arrangement not part of the present invention integrates a novel nasal pulse oximeter probe with a nasal cannula. Another particular object of the arrangement is to integrate a pulse oximeter probe with either a self-container breath apparatus (SCBA) or with the regulator of a self-contained underwater breathing apparatus (SCUBA). Blood oxygen measurements obtained by the so integrated pulse oximeter probe then are used to regulate the percentage oxygen in the supply of gas to the user, and/or to regulate the flow rate to the user upon inhalation. In the case of a SCBA apparatus that is combined with a pulse oximeter probe and oximeter, where that mask is worn in environments with toxic or noxious atmospheres, a critical role of the sensor is to indicate to the user when they are becoming hypoxemic, i.e. when there are potentially dangerous gases leaking into the mask. In the case of a SCUBA apparatus that is combined with a pulse oximeter probe and oximeter, for any dive the oximeter can provide information related to the formation of an air embolus. For deep dives, where specialty mixed gases are used and oxygen concentration in such mixtures are actually reduced from its concentration in air, the oximeter data on blood oxygen saturation provides a warning of current or pending hypoxemia. When further combined with a control to adjust the relative concentration of oxygen to other gases, this device serves to increase the relative oxygen concentration delivered to the diver when the oximeter data trend so indicates the need.

Still another arrangement not part of the present invention provides a pulse oximeter probe and a supply of oxygen or oxygen-rich air, in combination, and functioning in concert with each other and with oximetry circuitry, such that the level and trend in blood oxygen saturation are determined by the pulse oximeter and changes in blood oxygen saturation that indicate a sufficient downtrend in the blood oxygenation status results in a local or remote alarm to alert the patient and/or others to the problem.

The foregoing has outlined some of the more pertinent objectives of the present invention. These objectives should be construed to be merely illustrative of some of the more prominent features and applications of the invention. Many other beneficial results can be attained by applying the disclosed invention in a different manner of modifying the invention as will be described.

It is to be understood that the foregoing general description and the following detailed description are exemplary and explanatory only and are not to be viewed as being restrictive of the present, as claimed. These and other objects, features and advantages of the present invention will become apparent after a review of the following detailed description of the disclosed embodiments and the appended claims.

### Brief Description of the Drawings

**Figure 1** illustrates a side view of a hook-shaped pulse oximeter probe showing a preferred positioning of a LED pad having two LED's and at least one photodiode detector according to the present invention. This probe is configured for positioning across the lip or cheek of a patient.
**Figures 2A-C** provide top, front, and cross-sectional views, respectively, of a pulse oximeter probe not part of the invention for positioning in the nares of a nose of a patient. Connecting wires are shown in schematic format, not to scale. **Figure 2D** provides an enlarged view of one area of Figure 2A. **Figure 2E** provides a perspective view of a protective sheath used to cover the pulse oximeter probe of this figure.
**Figure 3A,B** depicts a side view and a top view of a pulse oximeter probe for positioning on the tongue of a patient.
**Figure 4** illustrates a perspective angled side view and an exploded frontal cross-sectional side view of a flat surfaced, elongated spring-loaded pulse oximeter probe showing the configuration of the LED's and the photodiode detector according an arrangement not part of the present invention.
**Figure 5** illustrates an internal view of the pulse oximeter sheath according to the present invention.
**Figure 6** illustrates a perspective angled side view of a flat surfaced, elongated spring-loaded pulse oximeter probe showing the features of the pulse oximeter sheath according to the present invention.
**Figure 7** is a flow chart showing one method utilized by the non-invasive vascular perfusion/resistance monitoring system to measure oxygen saturation values according to an arrangement not part of the present invention.
**Figures 8A and 8B** provide front and side views, respectively of a novel combined nasal pulse oximeter probe/oxygen cannula not part of the invention.
**Figure 9A** provides a side view of a typical self-contained underwater breathing ("SCUBA") apparatus typical of the prior art apparatuses. **Figure 9B** provides a side view of a SCUBA apparatus of the same configuration; which is not part of the invention, with a cross-lip pulse oximeter sensor added to this apparatus.
**Figure 10** provides a schematic flow diagram not part of the invention for control signal transmission from a pulse oximeter sensor to a control circuit to an oxygen control valve being controlled by data output from the sensor, where the control valve regulates oxygen to a patient wearing the sensor.

### Detailed Descriptions of Embodiments

The present invention discloses pulse oximeter probes for use with pulse oximeter systems in general.

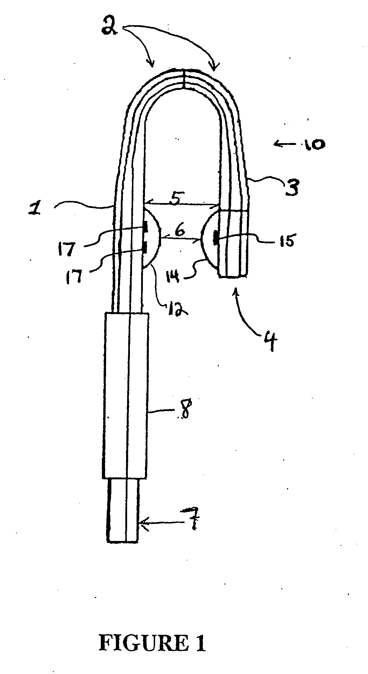

Figure 1 illustrates a pulse oximeter probe, **10**, of the invention, that is configured for placement with a section of the probe placed inside the mouth for measurement across the vascularized tissue of the lip or cheek. The probe, **10**, as depicted in Figure **1**, is comprised of a frame that is generally hook-shaped, having a longer proximal arm, **1**, a curved bridging section, **2,** and a shorter distal arm, **3**, the latter arm having a free end, **4**, that enters the mouth when in use. At least one portion of the proximal arm, **1**, is positioned at a specified distance, **5**, from an opposing portion of the distal arm, **2**, to provide a distance between the closest points of the two opposing arms, **6**, that accommodates the thicknesses of the lips and/or cheeks of a desired range of patients. As shown in Figure 1, the opposing portions of the proximal arm, **1**, and the distal arm, **2**, that are the specified distance, **5**, represents most of the lengths of these arms. In other embodiments of this probe, a smaller percentage of the total span of opposing arm sections may be set to such specified distance.

The probe may be used once and disposed, or may be repeatedly used on different patients. Preferably, the probe frame is constructed of metals, plastics, and other materials subjectable to repeated cleaning with standard antiseptic solutions, or sterilizable by other means. A cable, **7**, houses conductors (not shown), such as but not limited to insulated electrical wires, that connect operative components further in the probe, **10**, with an oximeter monitor (not shown). A boot, **8**, connects the cable, **7**, to the proximal arm, **1**, of the hook-shaped frame of the probe **10**. Preferably the cable, **7**, is flexible. The boot, **8**, primarily serves to connect the cable, **7**, with the frame, and secondarily to provide a handle with which the patient or attendant grip the probe. In other designs of the lip/cheek probe, a boot is not required where a direct connection is made between the cable and the frame of the probe.

In the embodiment depicted in Figure 1, the probe **10** comprises two LEDs **17** within an LED pad, **12**, and one photodiode detector **15** within a photodetector pad, **14**. These are the operative components of the probe, **10**, and are connected to a monitor system (not shown) by conductors (not shown) to transmit electrical signals. It is noted that although the two LEDs, **17**, are shown as two physically separate components in Figure 1, when present on a circuit board, typical LEDs are very small (about the size of a pencil point), yet discrete components. Thus, the two LEDs alternately can be represented as both being present on a single structure in other figures.

Each probe **10** is sized appropriately to be placed with the open end, **4**, inside a patient's mouth, so that the distance, **6**, between the LED pad, **12**, and the photodetector pad, **1**4, conforms to the thickness of the lip or cheek vascular bed of the patient. It is noted that Figure 1 is not accurately drawn to scale, and given the true small size of the pads **12** and **14**, the actual difference between the distances **5** and **6** is less than about 0.0127 m (0.5 inches). In practice, one probe 10P (not shown) is sized for the average pediatric patient, age 6-12, and another probe 10A (not shown) is sized for the average adult patient.

The embodiment depicted in Figure 1 has the light-sensing device, such as the photodiode detector, **15**, positioned in the mouth, on the side with the open end, **4,** as shown in Figure 1. Having the light-sensing device on the inside side of the cheek or lip minimizes erroneous readings due to interference from ambient light sources. Such light sources are much more likely to affect a light-sensing source that is positioned on the outside side of the cheek or lip. However, having the light-sensing source positioned on the outside side of the cheek or lip is within the scope of the invention.

Individual conductors provide electrical signals that power the LEDs **17.** Other conductors carry signals from the photodetector, **15.** Optionally, other sensors, such as for temperature, may be added to the probe, **10**, and have individual conductors for them also passing in the cable, **4,** to the frame of the probe, **10**. The probe, **10**, is used to generate data used to calculate oxygen saturation, pulse shape, blood pressure measurement (by measurement of pulse transit time to a second site), and any combination of these.

The bridging section, **2**, flexes to permit conformance to a range of tissue thicknesses greater than the nominal unflexed spans, as depicted by distances **5** and **6**. The probe in Figure 1 preferably is constructed of materials, such as nylon plastic, that impart a resiliency such that after bending, the probe returns substantially to its original shape. This resiliency allows the angular and dimensional relationships between the light-generating sources and the opposingly placed light detector to remain substantially consistent. Thus, the material for one embodiment of the probe has a degree of flexibility, and the material has sufficient memory to substantially return to its original shape after a normal flexion. This allows for standard use that may involve placement across lip and cheek tissue sections having different thicknesses, and movement across a thicker tissue section to ultimate placement at a thinner section.

For instance, in one embodiment of this configuration, the body of the probe, **10**, is made of nylon plastic. The flexibility of the bridging section, **2**, the proximal arm, **1**, and the distal arm, **3**, is such that less than 5 grams of force deflects the open end, **4**, in one direction or the other (toward or away from the opposing section) by about 0.0015875 m (1/16 inch) The force required increases logarithmically, such that to move the open end, **4**, outwardly 0.00635 m (0.25 inch) required between about 1,250 to 1,550 grams of force, and the force required to move the open end, **4**, inwardly (toward the opposing section) required between about 2,200 to 2,700 grams of force. After such forces the nylon material demonstrated memory, returning to within 0.0015875 m (1/16 inch) of the original position, thus demonstrating a resilient quality to the structure of the probe.

In addition, the material of each of the LED pad, **12**, and the photodetector pad, **14**, deflects upon application of pressure from adjacent tissue by up to about 0.00127 m (0.050 inch). Thus, the overall flexibility is sufficient to accommodate a wide range of sizes of cheek and lip sections, which the axis of light transmission from the LEDs is reliably aligned to the photodiode or other light sensor. While not being bound to a particular theory, it is believed that maintaining appropriately narrow alignment of these elements improves the reliability, precision and accuracy of the signals from the probe.

More flexible probes are alternate embodiments of the present invention. For instance, the structural material and thickness is adjustable such that only between about 150 to 1,250 grams of force moves the open end, **4**, outwardly 0.00635 m (0.25 inch), and between about 200 to 2,200 grams of force the force moves the open end, **4**, inwardly (toward the opposing section).

Less flexible probes also are alternate embodiments of the present invention. For instance, the structural material and thickness is adjustable such that between about 1,550 to 3,500 grams of force moves the open end, **4**, outwardly 0.00635 m (0.25 inch), and between about 2,700 to 5,000 grams of force the force moves the open end, **4**, inwardly (toward the opposing section). Alternately, in a more rigid probe, the structural material and thickness is adjustable such that between about 3,500 to 5,500 grams of force moves the open end, **4**, outwardly 0.00635 m (0.25 inch) and between about 5,000 to 8,000 grams of force the force moves the open end, **4**, inwardly (toward the opposing section). Such probes are made of metals or polymer composite materials. The resiliency is expected to vary inversely, roughly, with the flexibility of probes of such alternative embodiments.

Although in Figure 1 the bridging section, **2**, is curved, other embodiments of this lip/cheek probe may have a bridging section of any shape and angle, so long as it spans a distance and connects the opposing sides upon which the operative components of the probe are placed.

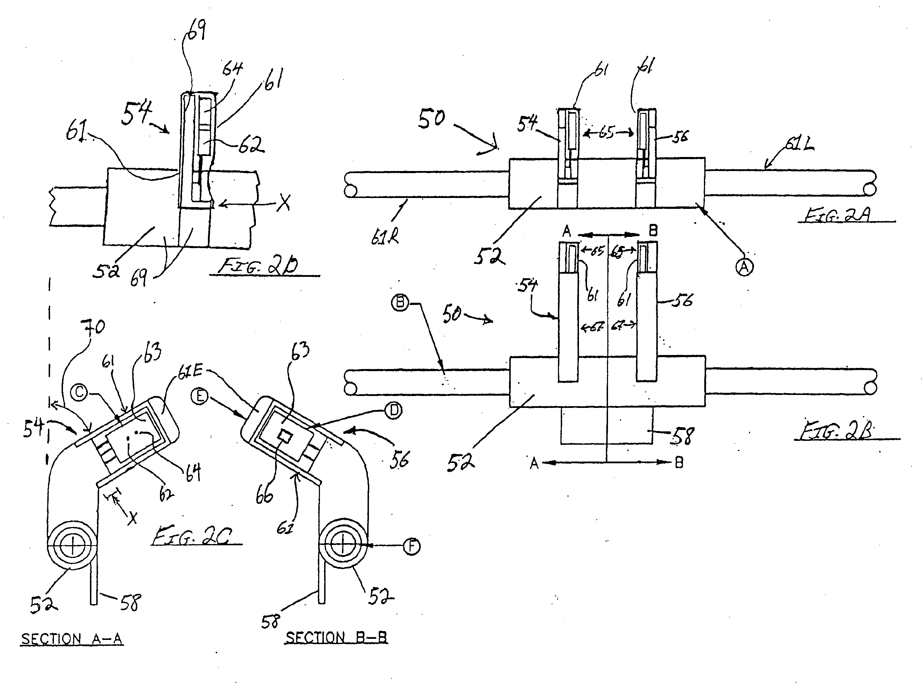

Figure 2A-C illustrates a second pulse oximeter probe, **50**, not part of of the invention, that is configured for placement inside the nostrils of the nose for measurement across the vascularized tissue of the nasal septum. Figure 2A is a top view, Figure 2B is a side view, and Figure 2C shows two cut-away views from a single mid-section line viewing opposite ends of the probe. From a main section, **52**, of a resilient plastic housing, extend two extensions, **54** and **56**, that are sized to enter the nares of the nose in similar fashion to a nasal cannula oxygen supply. These extensions, **54** and **56**, are flattened in one dimension, as depicted in Figures 2A and 2B, and are shown angled at about 60 degrees in a second dimension, as viewed in Figure 2C. This angle of inflection, **70**, is properly drawn from a line drawn from one edge of the main section, **52**. As discussed in greater detail below, the 60 degrees as depicted is not within the preferred range.

Two general approaches are used to protect the components of the pulse oximeter probe, **50**, from moisture and contamination. First, a clear plastic covering, shown as **61** in Figures 2A and 2B, and better viewed in Figures 2C and 2D, is placed over, to cover, each distal half of the two extensions, **54** and **56**. It is noted that in the arrangement shown, the molded outer shell, **69**, that forms and covers the main section, **52**, also covers the approximately proximal half of the two extensions, **54** and **56**, and the outer side of the upper, or distal halves of these extensions, but does not cover the front and rear sides, nor the inner sides, **65**, of these extensions. To cover these exposed sides, a clear plastic covering, **61**, is constructed, fitted over, and adhered to the existing components to form an integral protective exterior surface with the molded outer shell, **69**. This is viewable in Figures 2C and 2D. Such plastic covering, **61**, typically is manufactured by heat sealing pre-cut and/or pre-formed pieces, such as a cylinder or tube of heat-shrink plastic, to form a fitted covering over the distal halves of extensions **54** and **56**. Then this is shrink-wrapped over the components of the distal half of the two extensions, **54** and **56**. As depicted in Figure 2A-D, after heat-shrinking a cylinder of heat-shrink plastic, **61**, over each of the two extensions, **54** and **56**, the distal end of this plastic is glued together to forms an end, **61E**, over the distal end of each of the two extensions, **54** and **56**. These ends, **61E**, are viewed in Figure 2C.

Also, typically these pieces of heat-shrink wrap plastic, **61**, are sized and positioned to extend onto the approximate bottom half of two extensions, **54** and **56**, by about 0.0015875 m (1/16 inch), to form an integral seal against moisture (this overlap is shown at arrow X in Figures 2C and 2D). (It is noted that neither these nor other figures are drawn to scale, nor do they provide consistent proportions from figure to figure). In particular, Figures 2C and 2D show that a plastic cover, **61**, is fitted over each of the circuit boards, **63**, that contain the LEDs **62** and **64**, located on extension **54**, and the photodetector, **66**, located on extension **56** (see Figure 2C for details of LEDs **62** and **64**, and photodetector **66**). The plastic covers, **61**, preferably do not interfere with light transmission in the critical wavelength ranges of the LEDs **62** and **64**. Apart from heat-shrink sealing, other means of attaching the plastic covers, **61**, to the extensions **54** and **56**, include, but are not limited to, sonic welding, spot gluing, hot gluing, press fitting, and other such methods of attachment, as are employed in the art, that are used to attach components of a medical device for entry into an orifice of a living subject. Also, other means of providing a protective covering, such as are known to those skilled in the art, may be used instead of the above-described approach.

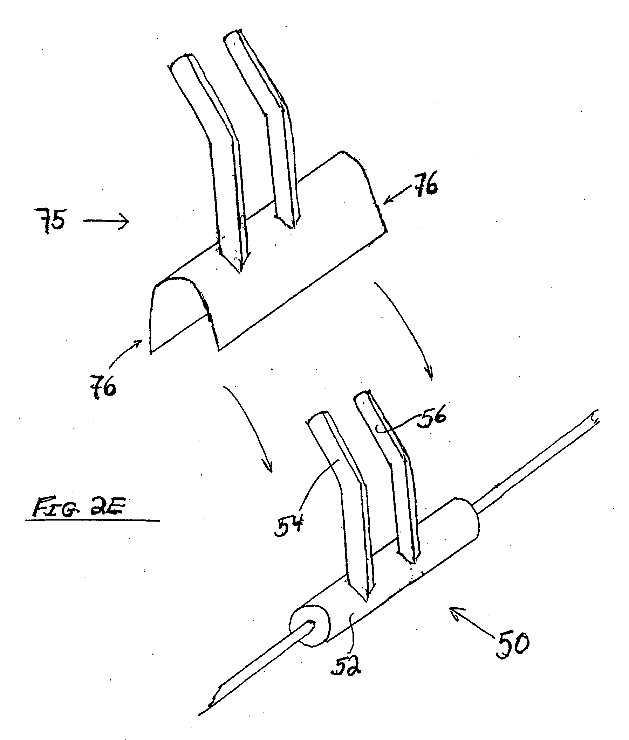

The above-described first protective approach is sufficient to prevent moisture and contamination of the components within the distal halves of the two extensions, **54** and **56**. A second approach, which is an optional and not required for the operation of the pulse oximeter probe, **50**, provides additional protection to this and other parts of the pulse oximeter probe, **50**. This is shown in Figure 2E. A protective sheath, **75**, of clear plastic, is dimensioned to slip over the entire two extensions, **54** and **56**, and then has flaps, **76**, that loosely cover the main section, **52**. The protective sheets are manufactured and priced so as to be disposable, so that after each use by a patient the protective sheath, **75**, is slipped off the pulse oximeter probe, **50**, and disposed of Then the exterior surfaces of the pulse oximeter probe, **50**, are wiped with alcohol or other suitable disinfectant. Then, prior to the next use, a new protective sheath, **75**, is slipped over the indicated parts of the pulse oximeter probe, **50**. Alternatively, the protective sheath, **75**, is made of a material that will withstand repeated rigorous disinfection procedures (such as steam autoclaving) without deformation or degradation, such as is known in the art, and such protective sheathes are used on numerous patients, with a disinfection process conducted between each use.

Preferably, the two extensions, **54** and **56**, are spaced apart from one another so that, upon insertion into the nostrils of a patient, the inner sides, **65**, of the extensions, **54** and **56**, fit snugly against the tissue of each side of the septum, to avoid interference from ambient lighting. Further, using the shrink-wrapping construction described above to cover the distal halves of the extensions **54** and **56**, and dimensioning the spacing between the extensions **54** and **56**, so as to fit snugly against the tissue of each side of the septum, these are found to fit without irritation, as from a rough or uneven surface. For example, without being limiting, when using heat sealing plastic as the covering, **61**, the thickness of this material, and any finish on the adjoining edge, will affect the extent of a sensible ridge at the junction of the covering, **61**, and the molded outer shell, **69**.

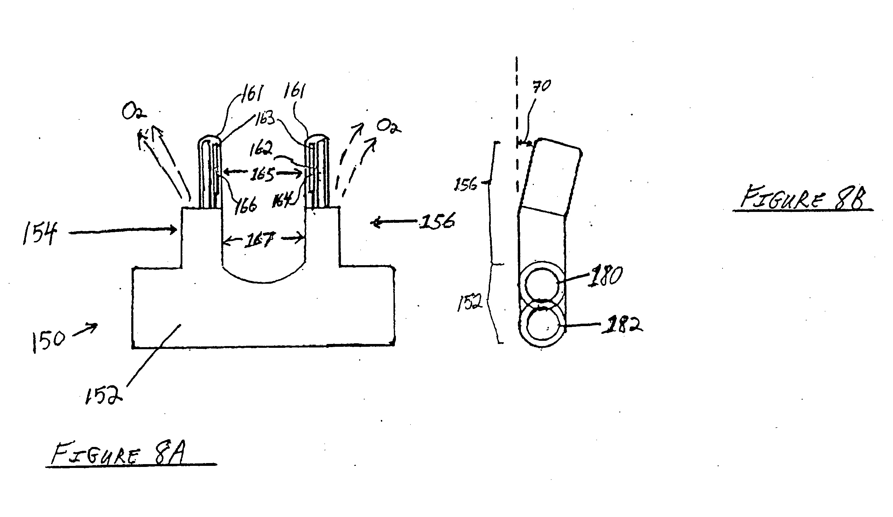

As to the specific area of the nasal septum that is preferred for use of a nasal pulse oximeter probe such as the one depicted in Figures 2A-D, it has been learned that the area of the nasal septum closest to the face (e.g., the proximal area of the middle alar), is more consistently vascularized and thereby provides more consistent and reliable signals than the areas more distal, i.e., the septum closer to the point of the nose. In particular, and more specifically, a highly vascularized region of the septum known alternately as Kiesselbach's plexus and Little's area, is a preferred target area for detection of blood oxygen saturation levels by a nasal pulse oximeter probe of the present arrangement In the particular device shown in Figures 2A-D, an angle of inflection, **70**, is shown between plastic housing, **52**, and the two extensions, **54** and **56**. This angle properly is measured as an interior (proximal) deviation from a straight line extended from the plastic housing, **52**. In preferred arrangement the angle of inflection, **70**, is between about 0 and about 33 degrees, in more preferred arrangements, the angle of inflection, **70**, is between about 10 and about 27 degrees, and in even more preferred arrangements, the angle of inflection, **70**, is between about 10 and about 20 degrees. In Figure 8B, the angle, **70**, is about 15 degrees. This angle has been found to provide superior results in testing. Therefore, the angle shown in Figure 2C, namely 60 degrees, is not a preferred angle of inflection.

Thus, in general, the two extensions, **54** and **56**, are angled so that upon insertion and proper placement into position in the nostrils, the LEDs **62** and **64**, located on extension **54**, emit light directed through a region that includes the preferred, proximal area of the nasal septum. Most preferably, the LEDs **62** and **64**, located on extension **54**, directed light exclusively through the highly vascularized region of the septum known alternately as Kiesselbach's plexus and Little's area.

In addition, a stabilizer, **58**, shown in Figure 2a as a flat plate flush with and extending downward from the inside edge of the lower plane of the extensions **54** and **56** (before the extensions angle inward, see Figure 2C), is designed to press against the area between the upper lip and nose to hold the desired position of the probe, **50**, and in particular the LEDs **62** and **64**, in relation to preferred, proximal area of the nasal septum. The stabilizer, **58**, although previously considered part of a preferred arrangement not a necessary component, has been found, on testing, to irritate many users, and to not be preferred. Additional means of stabilizing the probe, **50**, such as elastic straps from any part of the device that span the head of the patient, may be employed with or separately from the stabilizer, **58**. Thus, in preferred arrangements, no stabilizer, **58**, is used, and the design of the device, as shown in other figures provided herein, with or without additional stabilizing means, are adequate to stabilize the probe, **50**, during normal wear.

As for the probe described above in Figure **1**, timed electrical impulses from a pulse oximeter monitor system pass through two wires (not shown) in cables **61R** and/or **61**L to produce the light from LEDs **62** and **64**. At least one photodetector, **66**, is positioned on extension **56** to face and oppose LEDs **62** and **64** of extension **54**. The photodetector **66**, which typically is a light-sensing photodiode, detects changes in the light emitted by the LEDs **62** and **64** as that light is differentially absorbed between and during pulses across the capillaries of the septum tissue between the two extensions, **54** and **56**. In one arrangement LED **62** emits light around 650-670 nm, and LED **64** emits light around 880-940 nm. The electrical impulses are timed to be offset from one another. The photodetector, **66**, detects the light passing through the septum of the nose, which is situated between extensions **54** and **56** when the probe **50** is in use. As discussed above, loss of signal through vascularized tissue such as the nasal septum is due both to background tissue absorption and the absorption by the blood in the arteries, which expands during a pulse. The signals from photodetector **66** pass through conductors (not shown) to the processor of the monitor system (not shown). The "signal" as used here, is meant to indicate the signal from a photodetector receiving light from one or more light sources of the pulse oximeter probe, which provides information about differential absorption of the light during different parts of the pulse. These signals are to be distinguished in this disclosure from signals (electrical impulses) that are sent to the light sources to emit light, and from control signals that are sent, in certain arrangements to control a valve to supply more or less gas to a system.

Cables **61R** and **61L** preferably form a loop that may lie above the ears of the patient, and join to form a single cable (not shown). This single cable preferably terminates in an electrical plug suited for insertion into a matching socket in the pulse oximeter monitor system (not shown). The single cable terminates by connecting to an adapter cable, which in turn connects to a socket in the pulse oximeter monitor system (not shown). In a typical application, the signals from the light-sensing photodetector, **66**, are ultimately received and processed by a general purpose computer or special purpose computer of the monitor system (not shown).

In a variation of the nasal probe, such as is exemplified in in Figure 2A-C, oxygen is delivered with the same device that also measures trans-septum arterial oxygen saturation. In another variation, the pulse oximeter sensor is independent of an oxygen cannula, and is a single-use unit. In yet another variation, the pulse oximeter sensor is independent of an oxygen cannula, and is re-usable and readily cleanable with appropriate antiseptic cleaning agents. Other variations within the scope of the invention described and pictured can be developed by those of ordinary skill in the art.

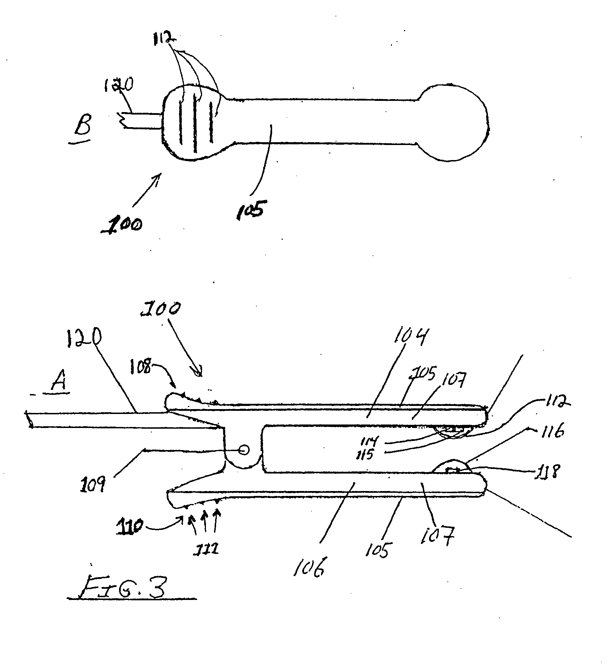

Figure 3 illustrates a third pulse oximeter probe, **100**, not part of the invention, that is configured for placement on the tongue of a patient for measurement across the vascularized tissue of the tongue. The probe, **100**, has two substantially flat opposing arms, **104** and **106**. A housing cover, **105**, is joined with a housing base, **107**, to form each of the two arms, **104** and **106**. At one end of each of the two arms, **104** and **106**, are finger pads, **108** and **110**, which in in Figure 3 are on the housing covers, **105**, and possess ridges, **111**, to improve the grip.

The arms, **104** and **106**, are tensioned to close against one another by a spring (not shown) which has a fulcrum at or near an axle, **109**, that hingedly connects the two arms, **104** and **106**, near one end. At or near the other end is an LED pad, **112**, on one arm, **104**. Within this pad, **112**, are two light generating sources, here shown as LEDs **114** and **115**. Opposite this housing, **112**, on arm **106**, is a photodetector pad, **116**. Within this pad, **116**, is at least one photodetector, **118**. Electrical wire conductors (not shown) connect the LEDs, **114** and **115**, and the photodetector, **118**, to a pulse oximeter monitor system (not shown), via a cable, **120**, passing from one end of the arm, **104**. The inner surfaces of the arms, **104** and **106**, in some variations of this probe are knobby or otherwise textured, especially around the LED pad, **112**, and the photodetector pad, **116**. This texturing is designed to better maintain a stable position of the probe, **100**, on the tongue without use of excessive pressure of the spring.

The photodetector **118**, which typically is a light-sensing photodiode, detects changes in the light emitted by the LEDs **114** and **115** as that light is differentially absorbed between and during pulses across the capillaries of the tongue tissue between the two arms, **104** and **106**. LED **114** emits light around 650-670 nm, and LED **115** emits light around 880-940 nm. The electrical impulses are timed to be offset from one another. The photodetector, **118**, detects the light passing through the tongue which is situated between the first housing, **112**, and the second housing, **116** of arms **104** and **106** when the probe **100** is in use. As discussed above, loss of signal through vascularized tissue such as the tongue is due both to background tissue absorption and the absorption by the blood in the arteries, which expands during a pulse. The signals from photodetector **118** pass through conductors (not shown) housed in cable **120** to the processor of the monitor system (not shown). Cable **120** preferably terminates in an electrical plug suited for insertion into a matching socket in the pulse oximeter monitor system (not shown). In another preferred arrangement cable **120** terminates by connecting to an adapter cable, which in turn connects to a socket in the pulse oximeter monitor system (not shown). In a typical application, the signals from the light-sensing photodetector, **118**, are ultimately received and processed by a general purpose computer or special purpose computer of the monitor system (not shown).

There are numerous means for hingedly joining the first arm and the second arm other than by an axle passing through the extensions of each arm (e.g., by axle **109**). Other means include hinges of various materials and designs as known in the art, co-fabrication of the arms with a thinner section of flexible plastic between the two arms at one end, and pins, screws, and other fasteners as are known to those skilled in the art.

Similarly, means for tensioning the first arm and the second arm, so as to properly maintain tension on a section of the tongue of a patient, can be effectuated by means other than the spring described above. Separate elastic bands may be attached or may surround the arms, such as by attaching to protrusions spaced appropriately along , the arms. Also, the natural flexibility and resilience of a co-fabricated structure comprising both arms connected by a section of resilient plastic can provide both the means for hinging and the means for tensioning. Such fabrications may be deemed suitable for disposable units.

It is noted that for this and other probes disclosed herein, a single source generating at least two different light frequencies may be utilized instead of LEDs. Alternately, more than two LEDs may be used, such as to generate light at more than two frequency bands, for instance to increase accuracy and/or detect other forms of hemoglobin. Also, light receiving sensors, or photodetectors, other than photodiodes may be used, and more than one such sensor may be used in a single probe.

The pulse oximeter probes, such as **10, 50**, and **100** as depicted and as used with the monitoring systems in the present invention, take measurements by transillumination as opposed to reflectance. This is the preferred configuration. However, for any of these probes, both the light-generating devices, and the photodetector devices, can be configured adjacent to one another, on one arm or extension, to measure reflectance of the tissue on the interior of the mouth (e.g., the cheek), the lip, the nasal septum, or the tongue.

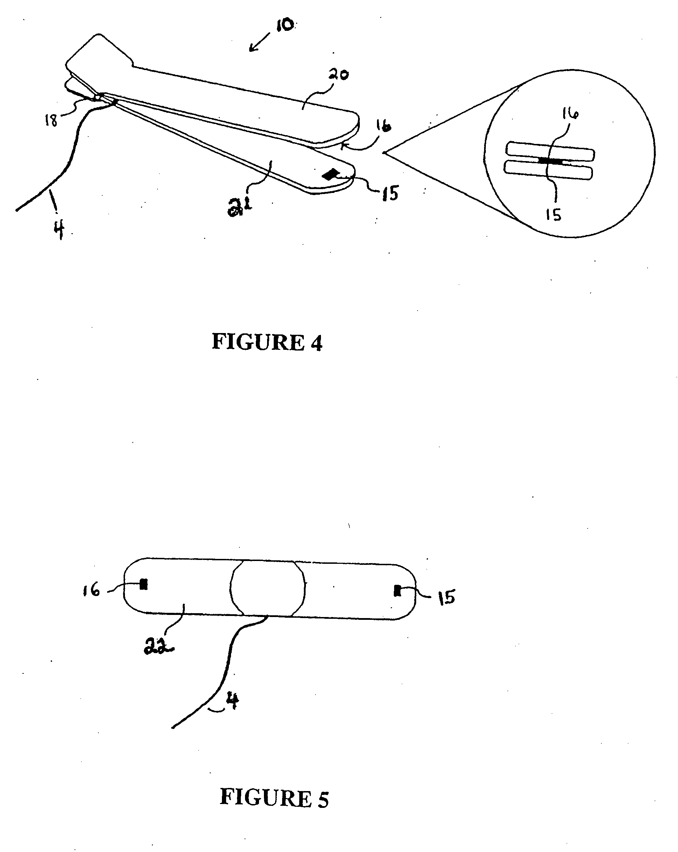

Figure 4 depicts another general configuration of an oximeter probe of not part of the present invention. This probe **10** can be dimensioned and configured to be expandable and tensioned to close by a spring, **18**. Near the distal, operative end of one substantially flattened side, **20**, is an LED array, **16**, and opposing it near the distal, operative end of the opposing substantially flattened side, **21**, is a light detecting sensor, preferably a photodiode, **15**. A cable, **4**, connects the LED array, **16**, and light detecting sensor to a pulse oximeter monitor system (shown in the magnified end view)). This pulse oximetry probe can be used to measure pulse-based differences in light absorbence across vascularized tissue of a patient in a number of locations, including but not limited to the cheek, the lip, the nasal alars (alar nari), the nasal septum, fingers, and toes.

By "substantially flattened" is meant that the height of the structure of a side is small relative to the greater of the length or width of that side. Preferably the ratio of the height to the greater of the length or width of a "substantially flattened" side is between about 0.2:1 and 0.001:1, more preferably this ratio is between about 0.02:1 and 0.005:1, and yet more preferably this ratio is between about 0.01:1 and 0.005:1. For greater applicability to typical physical requirements in use, each side also is substantially longer than wide. By "substantially longer than wide" is meant that the width of the structure of a side is small relative to the length of that side. Preferably the ratio of the width to the length of a side described as "substantially longer than wide" is between about 0.7:1 and 0.02:1, more preferably this ratio is between about 0.025:1 and 0.05:1, and yet more preferably this ratio is between about 0.025:1 and 0.1:1. At a minimum, with regard to nasal pulse oximeter probes the key functional attributes of extensions that are substantially flattened and/or substantially wide, as used herein, is that the width of such extensions is sufficient to house the components (i.e., circuit boards bearing LEDs and photodetectors, or the LEDs and photodetectors themselves), and the length of such extension is sufficiently long to provide the LEDs and photodetectors on opposite sides of a desired region of vascularized tissue. The same functional logic applies to other sensors disclosed and claimed herein.

Also, it is noted that in place of the spring, **18**, any hinging means as known in the art can be used. Such hinging means may include a raised section along or separate from the sides, such that a fixed space is created at the point of the hinging means. This would obviate the need for a bend in the sides at the spring, **18**, as shown in Figure 4 (which is required in Figure 4 to lever open the operative ends). These substantially flattened probes are configured such that the inner faces of both sides substantially oppose each other and, based on the spacing and configuration of the hinging means, are sufficiently separable to widen to encompass a desired tissue to be monitored for blood oxygen saturation between the light emitting structure and the light detecting structure at the operative end. It is noted that these structure may each be enclosed in a pad, or may not be so enclosed. As for other probes disclosed herein, a monitoring system connected to the probe modulates light signal production and receives signals of light detected by at least one light-detecting structure positioned at the operative end of one of the sides, such as **20** or **21** in Figure 4. Typically a pulse oximeter monitoring system includes, or can be connected to, a video monitor that provides graphical and numerical output from the signals received from the photodetector, which are algorithmically processed by a special-purpose (or general-purpose) computing component in the monitoring system.

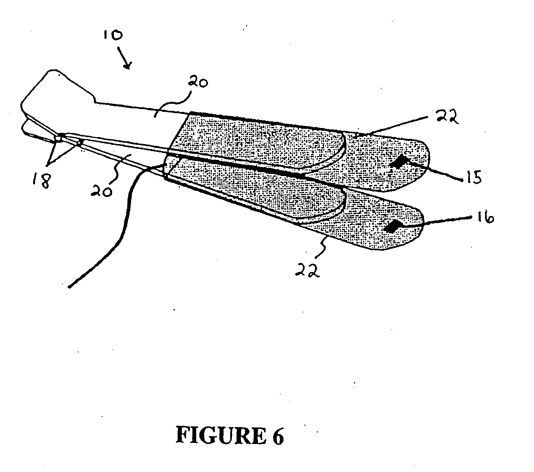

Also, the above substantially flattened sides with hinging means may be produced without light-emitting and light-detecting structures, and sleeves, such as described below, bearing such structures, would then be slipped over the sides to yield an operable oximeter probe. For instance, as shown in Figures 4 and 6, a probe **10** with a flat surface **20** is suitable for to receive a flexible sleeve, **22**, that bears an LED array, **16**, and light detecting sensor, preferably a photodiode, **15**. This slips over the flat-surfaced structure, as shown about halfway on in Figure 6. As for the probe in Figure 4, a cable, **4**, connects the LED array, **16**, and light detecting sensor to a pulse oximeter monitor system (not shown).

In operation, the devices depicted in Figures 4-6 are placed around a finger, earlobe, or other extremity in order to obtain data.

Thus, another aspect of the present invention is a disposable sleeve that fits over any of the pulse oximeter probes disclosed and claimed herein, and over conventional probes. A sleeve is constructed of a flexible material, and is relatively thin, in the general range of *0.000127 0.00127m* (0.005 to 0.050 inches) thick, more preferably in the range of *0.000254-0.000381m* (0.010 to 0.025 inches ) thick, and most preferably in the range of 0.000254-0.000635m (0.010 to 0.015 inches) thick. The sleeve is manufactured to slide over the major structural features of the probe to provide a barrier to reduce the chance of contamination from one patient to a second patient using the same probe. An example of such sleeve is shown in Figure 5, and its implementation over a probe is shown in Figure 6. In this case the sleeve is constructed to include the light generating and the light sensing features, and associated conductors. However, in other embodiments of the sleeve, such features are on the major structural features, whether frames, arms, etc., and the sleeve slides over such features, and at least in the areas of such light producing and light sensing features the sleeve is highly transparent to the critical wavelengths used by the pulse oximeter. The sleeves cover both arms, or extensions, of probes having two distinct arms. Preferably a continuously integral section of the sleeve joins the sleeve sections that cover both arms or extensions of the probe, in order to, inter alia, protect the intervening parts of the probe. For a probe such as the probe of Figure 1, the sleeve is configured to the shape of the probe and slides over starting at the end, **4**, of the arm, **3**, and covers up to, and preferably including, the boot, **8**.

In some sleeve embodiments, a stretchable aspect of one or more parts, or of the entire sleeve, stretches over a protuberance or other prominence at one or more parts of the major structural features over which the sleeve is sliding, and improves the fit of the sleeve. This also better assures that the sleeve does not slide off the probe during normal uses. Alternate means to secure the sleeve onto the probe such as are known to those of skill in the art may be used. The sleeves themselves can be disposable; however, the sleeves also can be made of easily sterilizable materials and be sterilized between uses.

The probes and the sleeve covers of the present invention are supplied as clean or as sterile, depending on the needs of the end user and the budgetary constraints of the end user. Clean but not sterile probes and sleeves will be less expensive, and may be suitable for many applications. Where there is an elevated risk of major harm from an infection, for instance in immunocompromised patients undergoing transplants with immunosuppressive drugs or undergoing chemotherapy, sterile probes would be more appropriate than merely clean probes. Many configurations of the probes are cleanable using alcohol and/or detergent/disinfectant solutions, and other configurations are disposable.

All of the above disclosed probes operate in a typical manner of a pulse oximeter, as described herein and in articles and patents, Each LED emits its specific frequency hundreds of times per second, and the absorption (or transmittance) readings by a sensor, such as a photodiode, are transmitted to a computer. There a software system performs averaging (optionally deleting outliers), and by differences in wavelengths' absorption or transmittance at the pulse peaks, determines arterial oxygen saturation. In a standard two-LED system, this is done by an algorithm that calculates the ratio of the peak absorbence at 650-670 nm divided by the base absorbence at this wavelength range, and compares this ratio to the peak absorbence at 880-940 nm to the base absorbence at the 880-940 nm range. The base absorbence reflects the non-pulse background absorbence by tissues other than the artery at maximum width during the pulse. This calculation provides an estimate of arterial oxygen saturation. A graph of the pulse surge, or shape, over time, also can be obtained.

All of the above-disclosed probes are expected to have significant use in the intensive care units, operating rooms, post-surgery recovery rooms, and in ambulance related situations where a patient in need of monitoring has few suitable monitoring sites. The size and shape of each probe will depend on whether the patient is an adult or child.

When two or more probes are used together, data from multiple probes is processed to provide continuous and simultaneous cross-site comparisons of the arterial blood oxygen saturation status at and comparisons between two or more tissue sites (and, as desired, blood pressure estimates based on transit time differences and/or other related parameters). The monitoring system receiving these signals includes at least one program containing computer software (not shown) comprising instructions which causes the computer processor to receive and calculate various oxygen saturation values. Optionally, the monitoring system may receive signals from separate probes or sensors to assess blood pressure values, which optionally may be compared (either simultaneously or separately) with blood pressure estimates based on signals received from each of the probes determining arterial blood oxygen saturation and vascular perfusion/resistance of a patient. Depending upon the software used, and the addition of separate blood pressure probes or sensors, the monitor may be used as a dual pulse oximeter, a saturation difference monitor, a transit time monitor, a periodic blood pressure monitor, or a noninvasive continuous blood pressure monitor. Specific examples are provided below that demonstrate a non-exclusive range of applications for the monitoring system which compares signals from a central source site (CSS) with signals from at least one advantageously positioned peripheral site (PS), as those terms are defined herein.

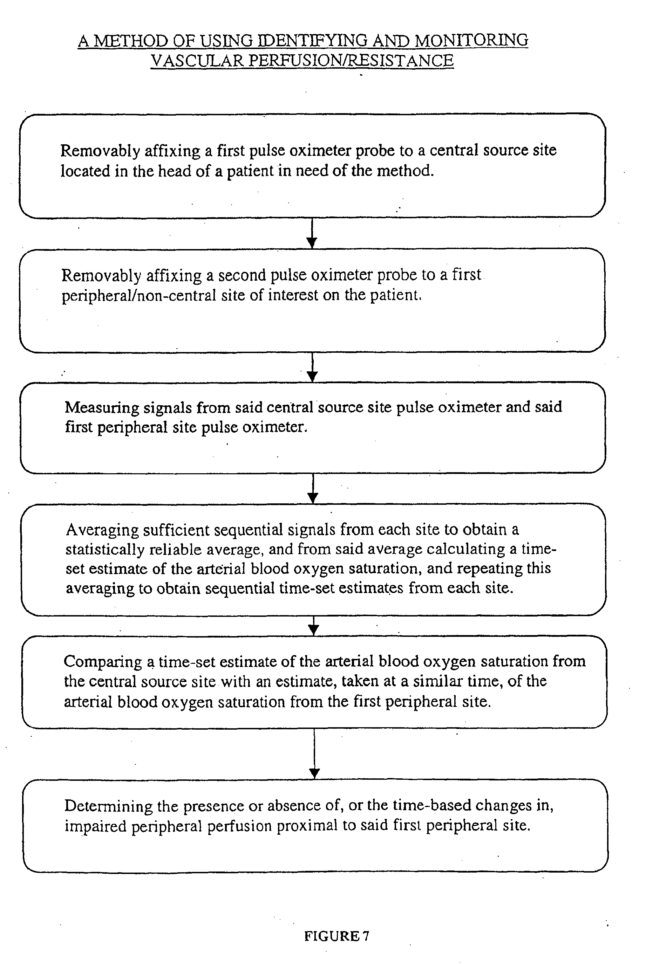

Figure 7 depicts the steps of a basic method not part of the invention using the monitor system that includes one probe positioned in a CSS, and one probe in a PS. A first pulse oximeter probe is removably affixed to a CSS in the head of the patient. This is most preferably any of the specially configured probes, or could be a conventional probe. A second pulse oximeter probe is removably affixed to a PS such as a finger or a toe. This can be any of the specially configured probes, or a conventional probe. The monitoring system is started, the LEDs or other light generating sources in the probes emit designated light at designated frequencies and periodicities, and signals from the CSS and from the PS are measured and transmitted to the monitoring system computer. Here, adjacent signals of the same type (wavelength and probe) are averaged to obtain a statistically reliable average. As appropriate based on the software program, certain outliers as may be caused by movement of the patient, light contamination from an outside source, etc., are eliminated from consideration. The averaging is repeated and the averaged values are compared based on the time sequencing of the respective averages. That is, average values from a specific time from the CSS probe are compared to average values from the same time span from the PS probe. The software calculates arterial blood oxygen saturation percentages based on the differential absorption of the different species of hemoglobin, and percent oxygen saturation at the CSS and the PS are compared. Based on criteria input into the monitoring system and reflected in the software's calculation, the presence or absence of impaired peripheral perfusion are shown as an output reading of the monitoring system. Alternatively, if impaired perfusion has already been established, the tracking of time-based changes in the saturation differences between the CSS and the PS are read out or charted.

The method shown in Figure 7 is conducted with an apparatus having the stated functional capabilities. Also, an oximeter monitoring system has the basic physical components that perform the required centralized functions, and which is attached to at least two oximeter probes to perform the above-described method.

Further, a variation of the method of Figure 7 is to have an additional PS probe, and compare not only the first PS probe to the CSS probe, but to also compare the first and second PS probes' signals to one another. This can, for instance, demonstrate impaired peripheral perfusion in one body area, but not in another body area or extremity.

The apparatuses, methods and systems of the present invention can be applied to both humans and animals, i.e., to living vertebrate organisms. Its application in human medicine (adult & pediatrics) would significantly improve the estimation of vascular perfusion or resistance by pulse oximetry; however, veterinary medicine also would greatly benefit from its use. This superior monitoring system would utilize at least two pulse oximeter probes, one of which is designed for use with a highly perfused central tissue, such as a lip, tongue, nares, cheek; and the other probe is designed for use to less perfused areas such as peripheral tissues, or any combination thereof.

The following specific examples are meant to be demonstrative, but not limiting, of the possible applications of the present invention.

### Example 1:

Data from a small number of volunteer subjects was obtained. This data provided preliminary support for the hypothesis that differences in CSS and PS estimates of arterial blood oxygen saturation levels can provide diagnostic information about the status of peripheral blood circulation. These data are summarized below.

All sets of data were taken three times, except that data for subjects 1 and 9 were only taken two times (duplicate data sets). Subjects 1-3 had no history of chronic obstructive pulmonary disease or other conditions that would be expected to cause lowered peripheral circulation. Except for one reading of 93% for subject 1, all estimates of arterial oxygen saturation were 95% or higher, and the PS (a finger, using a standard commercial probe) readings were within two percentage points of either CSS sites (lip and cheek). For the data set in which subject 1's cheek probe reading was 93%, the lip reading was 98% and the finger reading was 96%. Overall, the data of subjects 1-3 suggest that in a healthy subject the CSS and PS readings taken at or near the same time will be relatively close, within about 5 percentage points or less, and all of the readings will be high.

Subject 4 had average readings at the PS finger site of 89%, and at the CSS cheek site, 88.7%, so these sites has essentially identical estimates. No signal was recorded at the lip CSS. Although there was no difference between the CSS cheek and the PS readings, the oxygen estimate was low and indicated a generalized problem.

Subject 5 had a PS average of 85%; the lip CSS average was 88.3%, and the cheek CSS average was 91.3%. The absolute levels are low, and the difference between CSS and PS values ranges from about 3 for the lip to about 6 for the cheek. This appears to suggest a peripheral circulation problem, and the low absolute levels indicate a generalized problem with oxygenation. This subject was known to have COPD.

Subjects 6-8 were known to have COPD. The average values for finger, lip and cheek were 85, 90, and 89, respectively for Subject 6. The 4-5% less percent saturation for the peripheral site supports the present hypothesis. Subject 7's finger data varied between 77-80% during the readings, and is considered unreliable. One of subject 8's data points for the finger was 79%, whereas the other two were 85%. This suggests that the 79% reading is erroneous. Disregarding this data point, Subject 8 had 85%, 87.3%, and 85.6% averages for the finger, lip and cheek sites, respectively. Here, all readings are fairly close, and the absolute values are alarmingly low. The data from this subject do not support the hypothesis; however the circulation for this subject may not be impaired peripherally. Further investigation can resolve this and other points.

Regarding the latter, subject 11's data was anomalous in that the finger site averaged 93.3%, whereas the lip and cheek sites averaged 90.7% and 86.7%, respectively. The reason for this is unknown; the data could be spurious or could indicate unusual circulation in a small percentage of the population. Individual differences in circulatory systems (based in part on genetics, and in part on non-genetically based embryological development, and on physical conditioning) may form the basis for such anomalies in a percentage of the population. Highly variable and incomplete data for Subjects 9 and 10 were considered to render the value of their data questionable, and those data were not analyzed.

Thus, this preliminary data provided some indication of differences between CSS and PS and differences between normal and circulation-compromised subjects. The data also supported the need to investigate broader populations with know circulatory conditions to develop more predictive guidelines for the probe data differences. Even with the limited data of this example, it is apparent that the comparison of CSS and PS sites can provide a useful assessment of the state of the circulatory system even where there is no major difference, and there is not a disease state presenting itself. That is, such results of roughly equivalent CSS and PS data at a high oxygen saturation level would support a conclusion that the peripheral circulation is not impaired.

### Example 2:

An elderly patient with relatively advanced diabetes comes in for monitoring of the status of perfusion in the right leg, which is diagnosed with severe atherosclerosis and related impaired vascular perfusion. A monitor is utilized, with one CSS probe measuring signals across the nasal septum, and a PS probe on the large toe of the right foot. A new medication is started, and ongoing weekly data from the monitor tracks the changes in perfusion in the right leg by comparing oxygen saturation values of the CSS probe with the values of the PS probe. Such data indicates the degree of effectiveness of the new medication.

### Example 3:

A critically burned patient is brought into an emergency room. As vital signs and assessment is taking place, a pulse oximeter probe as shown in Figure 1 is placed into the patient's mouth to read cheek tissue as a central site source, and a pulse oximeter probe as shown in Figure 4 is placed at each of the patient's large toes. Within less than one minute, the monitor indicates below normal blood perfusion in the right leg, based on the signals from the probe placed on the right toe, compared to the central source site and the left toe probe. A probe is placed on a right finger, and this provides comparable data to the left toe. The attending physician is able to surmise that an injury or disease condition is adversely affecting perfusion in the right leg, orders more detailed testing, and increases the percent oxygen on the respirator to counter the low oxygen in the affected leg. The monitoring system tracks changes in the oxygen saturation values of blood in the right toe as this initial treatment has an effect.

### Example 4:

A patient suspected of having Chronic Pulmonary Obstructive Disease is admitted to an emergency room with breathing difficulties. The patient also reports pain in both legs after involved in a minor traffic accident, which is the immediate cause of admission. Minor bruising is apparent on the front of the left leg. Along with other tests and monitoring, a pulse oximeter monitor is utilized, with on CSS probe on the nares of the nose, and a PS probe on the large toe of each of both feet. Alarmingly, the CSS probe estimates that the arterial oxygen saturation at the CSS site is below about 85 percent, indicating hypoxia. The pulse oximeter monitor in both PS sites estimates even lower oxygen saturation, by about 5 percent, compared to the CSS site. There is no response to bronchodilator therapy, and the chest x-ray shows moderate fibrosis, and no attenuated vessels or hyperinflation. The initial diagnosis, aided by the pulse oximetry data, is bronchial COPD. Oxygen therapy is provided, and the pulse oximetry data is utilized to monitor increases in blood oxygen saturation both at the CSS and PS sites.

It is noted that the following paragraphs, through and including Example 5, describe arrangement which combine, preferably integrally, a pulse oximeter probe with a nasal cannula through which is delivered a supply of oxygen or oxygen-rich air. In some of these arrangements the combined pulse oximeter sensor/nasal cannula with oximeter is used to monitor and provide information regarding the oxygen saturation status, using data obtained from the sensor through the tissue of the nasal septum, to the user of the device, to caretakers of that user, and/or to a remote station that utilizes the information. For instance, the user can view current and/or historical trend data and manually adjust the flow rate of the oxygen or oxygen-rich air accordingly. Alternately, a user of said combined pulse oximeter sensor/nasal cannula with oximeter, in advance of a period of expected increased exertion, may increase the flow rate of his/her auxiliary oxygen supply. Then, during such exertion, such user refers to the oximeter data output and further adjusts the flow rate as needed to attain or remain within a desired range of blood oxygen saturation as indicated by the data output from the oximeter.

In other arrangements, the combined pulse oximeter sensor/nasal cannula is used in further combination with a central processing unit that sends signals to automatically adjust the flow rate of the oxygen or oxygen-rich air to the use. For instance, and not to be limiting, during more strenuous exertion, arterial blood oxygen saturation of a person needing oxygen supplementation therapy is expected to decline appreciably. In such circumstance, this drop in oxygen saturation is detected by the pulse oximeter probe, the trend data is analyzed by a program in the central processing unit, and a signal is sent to a valving mechanism that results in a greater oxygen flow directed through the user's cannula. A feedback loop, the data from the nasal pulse oximeter going to the central processing unit monitoring system, subsequently decreases the flow when the data indicates arterial blood oxygen saturation has exceeded a designated percentage. By such feedback loop approach, the oxygen delivered via the nasal cannula is better optimized for actual physical exertion and/or changing metabolic requirements.

In other arrangements, which are preferred in certain applications, the use of data from the nasal pulse oximeter to regulate oxygen flow to the nasal cannula is combined with other approaches to conserve oxygen, which include, but are not limited to:
1. detection of the inhalation phase of the respiration cycle to provide the oxygen (or oxygen-enriched gas) only during inhalation (or a key segment of the inhalation, i.e., the initial 2/3 of the inhalation);
2. providing oxygen every other breath; and
3. providing greater volume and/or flow rate at key part(s) of inhalation cycle (i.e., increased "shot size").

In yet other arrangements, are which are preferred in certain applications, the data from the nasal pulse oximeter is combined with data collection of other parameters. For instance, in studying sleep disorders, a number of parameters are measured, for instance, pattern or dynamics of respiration (flow rate, inhale/exhale over time cycles), pulse rate, etc. The use of the combined nasal pulse oximeter probe is combined with other monitoring sensors at the nose that detect, for instance, but not to be limiting, air flow and air pressure, such as during sleep, to analyze an individual's sleep disorder, such as sleep apnea.

Thus, when blood saturation information from the so-combined pulse oximeter probe is processed over time, and when trends are detected in the oximeter probe data processor that indicate a need for more or for less oxygen to the patient, based on, respectively, lower or higher blood oxygen saturation readings than a desired range, one or more outcomes result. As noted above, one outcome is to automatically adjust the supply of oxygen or oxygen-rich air to provide a needed increase (if readings were trending lower) or a decrease (if readings were trending higher, above a desired range, and conservation of the supply were desired) of that supply. Another outcome is to provide an alarm signal (audible, flashing, etc.) locally, for recognition by the patient or a nearby attendant. Yet another possible outcome is to provide a remote alarm, such as by cellular telephone transmission, to a physician's office, an ambulance service or hospital, etc.

Further, it is noted that there exist in presently used devices other approaches to conserving the supply of oxygen or oxygen-rich air. One commonly used approach, often referred to a the "pulse-dose" system, delivers oxygen to the patient by detecting the patient's inspiratory effort and providing gas flow during the initial portion of inspiration. This method is reported to reduce the amount of oxygen needed by 50 to 85% (compared to continuous flow) and significantly reduces the cost, the supplies needed, and the limitations on mobility caused by a limited oxygen supply.

For example, as the patient initiates a breath, the cannula tip senses the flow, a solenoid valve opens, and a burst of oxygen is rapidly delivered to the patient. The size of the burst or flow varies among different manufacturers. Commonly, the pulsed-dose system takes the place of a flow meter during oxygen therapy and is attached to a 3.44.10⁵ Pa (50 PSI) gas source. In most devices the patient or operator can choose the gas flow rate and the mode of operation (either pulse or continuous flow). Typically, a battery-powered fluidic valve is attached to a gaseous or liquid oxygen supply to operate the system.

In addition, other approaches are used to further reduce oxygen usage when using the pulse-demand system. One such approach is to reduce the dose of oxygen delivered to the patient during each pulsation. Another approach, in combination or independently of the last one, is to deliver a burst only on the second or third breath instead of every breath. In addition, the size of the oxygen pulse dose will change with the flow setting with increases in flow delivering larger doses of oxygen and vice versa.

It is noted that potential problems encountered when using the pulse-demand system include: no oxygen flow from the device; and decreased oxygen saturations in the patient. If no oxygen flow is detected, then possible causes include a depletion of the gas supply, an obstruction or disconnection of the connecting tubing, or, critically for a pulse-demand system, an inability of the device to detect the patient's effort to breath. If the device cannot detect the patient's inspiratory effort, the sensitivity will need to be increased or the nasal cannula will need to be repositioned in the nares.

A decrease in the patient's oxygen saturation should always be a cause for alarm and may indicate a change in the patient's medical status, tachypnea, or a failure in the device. In any case, a backup system should be available in order to verify whether the problem is with the device or with the patient.

Thus, although in common use, the limitations of many pulse-dose systems are: relatively high cost of the system; technical problems may be associated with such a complicated device (including disconnections, improper placement of the device, and a possible device failure); lack of accommodation for an increased need during exercise, stress, illness, etc. and variable operation of the device if not properly set up.

Variations on the pulse-dose system include delivering oxygen to the patient at the leading edge of inspiration. This allows oxygen to be supplied exactly when needed. Thus, when the patient inhales, a relatively higher quantity of oxygen is delivered for travel deep into the lungs, increasing the probability of greater utilization and uptake in the red blood cells in the person's bloodstream. Other variations on the pulse-dose system are known in the art.

The present invention is used independently of the pulse-dose system, or, alternatively, in conjunction with such system, to conserve the supply of oxygen or oxygen-rich gas, and to better adjust the supply to the actual demands of the patient/user as that person's physical activities and demands vary over time. In particularly preferred arrangement, the pulse oximeter probe that is in combination with an outlet (e.g., the end of a cannula) of the supply of oxygen or oxygen-rich gas is fashioned to be integral with, or securely fastened to, that outlet. This provides greater surety of signals and proper insertion of the outlet. For instance, when the pulse oximeter probe is integral with the nasal cannula, if the probe and device are accidentally moved from their proper location (e.g., entrance of nose, or mouth), then the oximeter readings (including pulse) will deviate sharply from normal. In such instance an alarm can be quickly sounded and the problem rapidly corrected. Thus, this provides a distinct advantage in comparison to peripheral probes, such as finger or toe probes.

Another aspect is adding as an additional sensor a capnography sensor (such as an infrared sensor) to estimate the concentration of carbon dioxide in the exhaled breath. This may be useful to detect more rapidly than pulse oximetry the failure of ventilation means (such as the wrong gas being provided to the patent), or carbon dioxide poisoning. Regarding the latter, the article entitled "Management of carbon monoxide poisoning using oxygen therapy" by TWL Mak, CW Kam, JPS Lai and CMC Tang, in Hong Kong Medicine Journal, Vol. 6, No. 1, March 2000 is instructive.

Also, as to the detection of a failure of ventilation means, when a combined nasal pulse oximeter sensor/cannula is attached to a pulse oximeter that is programmed to distinguish normal from abnormal pulse ranges, and when the combined nasal pulse oximeter sensor/cannula falls away from the user's nose (e.g., by accident during sleep or sedation, etc.), an alarm can be quickly provided based on the lack of pulse in the proper range. In this way the combined nasal pulse oximeter sensor/cannula more rapidly detects a loss of supplemental oxygen more rapidly than typical capnography detectors as to this reason for loss of ventilation.

Thus, the following examples are to be understood to be usable independently or in combination with the above described other approaches to conserving the supply of oxygen or oxygen-rich gas, and/or with other approaches known in the art but not described above, including those referred to in references cited herein.

### Example 5:

Figure 8 depicts one arrangement not part of the invention of a nasal oximeter probe, such as depicted in Figures 2A-D, in which the oximeter function and hardware are combined and integral with a cannula to supply oxygen (or oxygen-rich air or other gas mixture) to via the nostrils of the patient. The device, 150, shown in Figures 8A, B is but one specific arrangement of a range of designs and combinations that include a pulse oximeter probe in combination with an outlet for oxygen or oxygen-rich gas to a person in need thereof. For instance, while in the present example a cannula (defined as "a tube for insertion into body cavities and ducts, as for drainage") is used within the nasal oximeter probe to conduct oxygen-rich air or other gas mixture into the nostrils of a patient, any of a range of different conduits can serve this purpose. As one example, not meant to be limiting, a passage can be formed by molding such passages within the structure of the nasal oximeter. Such passages themselves can serve to conduct oxygen-rich air or other gas mixture into the nostrils of a patient. Alternately, these passages can be sized and configured to allow cannula tubing to be inserted through such passages, to provide for relatively easy assembly with standard cannula tubing which is common with standard regulators and tanks. Thus, the term "passage" is taken to mean any physical structure, now or later know to those of skill in the art, that provides for the physical containment of a gas that is being directed through such structure. Common forms of passages include cannula tubing, standard plastic tubing, and the continuous voids in a molded nasal pulse oximeter through which a gas may pass without loss from seams, etc. in the voids.

Figure 8A is a front view, and Figure 8B is a side view of the combined, or integral, nasal probe/cannula, **150**. From a resilient plastic housing, **152**, protrude two extensions, **154** and **156**, that are sized to enter the nares of the nose. Preferably, the lateral cross-sectional surface area of each of these extensions, **154** and **156**, is not greater than 50 percent of the opening cross-section area of a nares at its widest opening, more preferably the device's inserted cross-sectional surface area is not greater than 35 percent of such opening area of a nares, and even more preferably, the device's inserted cross-sectional surface area is between about 20 and about 35 percent of such opening area of a nares. At the end of these extensions, **154** and **156**, which preferably are of molded plastic and integral with the major portion, the plastic housing, **152**, are inserted two circuit boards, **163**, one containing two light-emitting diodes, **162** and **164** (shown here on extension **156**) and the other containing a photodetector, **166** (shown here on extension **154**).

In preferred arrangement, the two extensions, **154** and **156**, are spaced apart from one another so as to fit snugly against the tissue of each side of the septum, to avoid interference from ambient lighting. Also, it is noted that clear plastic covers, **161**, are placed over the molded plastic frame that forms the extensions **154** and **156** in Figures 8A-B. These plastic covers typically are heat-sealed over the LEDs **162** and **164** and photodetector **166**. Typically, the sides, **165**, of the clear plastic covers, **161**, that are facing or contacting the nasal septum (not shown) are aligned with the inside sides, **167**, of the extensions **154** and **156**, so as to fit snugly against the tissue of each side of the septum, without irritation, as from a rough or uneven surface. As noted in more detail elsewhere, the covers, **161**, preferably have inner faces co-planar with the inner faces of the two extensions, **154** and **156**. This is to ensure a comfortable fit, good data since ambient light is lessened, and no necrosis of the tissue being contacted. It is preferred that the two extensions, **154** and **156**, deflect from the septum wall due to flexibility of the structures themselves, **154** and **156**. This is particularly helpful when a patient has a septum wider than the spacing between the inner sides, **165**.

Further as to the plastic covers, **161**, one is fitted over each of the structures, **163**, that contain the LEDs **162** and **164**, located on extension **156**, and the photodetector **166**, located on extension **154**. The plastic covers, **161**, preferably do not interfere with light transmission. Apart from heat-shrink sealing, other means of attaching the plastic covers, **161**, to the extensions **154** and **156**, include, but are not limited to, sonic welding, spot gluing, hot gluing, press fitting, and other such methods of attachment, as are employed in the art, that are used to attach components of a medical device for entry into an orifice of a living subject. In general, the combined nasal pulse oximeter probe/cannula devices are designed to be disposable, due to problems associated with cleaning between uses. Appropriate plastics, components and construction are employed so as to allow an appropriate level of sterilization of such devices between uses.

As for the nasal pulse oximeter probe depicted in Figures 2A-D, two extensions, **154** and **156**, extend from a main section, **152**, of a resilient plastic housing. These two extensions, **154** and **156**, are sized to enter the nares of the nose in similar fashion to a nasal cannula oxygen supply. These extensions, **154** and **156**, are flattened in one dimension, as depicted in Figures 8A and 8B, and are shown angled at about 15 degrees in a second dimension, as viewed in Figure 8B. This angle of inflection, **170**, is properly drawn from a line drawn from one edge of the main section, **152**.

The first approach described above for the nasal oximeter probe in Figures 2A-D is used to protect the components of the combined nasal pulse oximeter probe, **150**, from moisture and contamination. A clear plastic covering, shown as **161** in Figures 8A, is placed over, to cover, each distal half of the two extensions, **154** and **156**. The molded shell, **169**, that forms and covers the main section, **152**, also covers the approximately proximal half of the two extensions, **154** and 156. Either this, or a separate resilient insert, provides a support for the upper, or distal halves of these extensions, but does not cover the front and rear sides, nor the inner sides, **165**, of these extensions. To cover these exposed sides, a clear plastic covering, **161**, is constructed, fitted over, and adhered to the existing components to form an integral protective exterior surface with the molded outer shell, **169**. Such plastic covering, **161**, typically is manufactured by heat sealing pre-cut and/or pre-formed pieces to form a fitted covering over the distal halves of extensions **154** and **156**. Then this is shrink-wrapped over the components of the distal half of the two extensions, **154** and **156**. The plastic covers, **161**, preferably do not interfere with light transmission in the critical wavelength ranges of the LEDs **162** and **164**. Apart from heat-shrink sealing, other means of attaching the plastic covers, 161, to the extensions **154** and **156**, include, but are not limited to, sonic welding, spot gluing, hot gluing, press fitting, and other such methods of attachment, as are employed in the art, that are used to attach components of a medical device for entry into an orifice of a living subject. Also, other means of providing a protective covering, such as are known to those skilled in the art, may be used instead of the above-described approach.

Preferably, the two extensions, **154** and **156**, are spaced apart from one another so that, upon insertion into the nostrils of a patient, the inner sides, 165, of the extensions, **154** and **156**, fit snugly against the tissue of each side of the septum, to avoid interference from ambient lighting. Further, using the shrink-wrapping construction described above to cover the distal halves of the extensions **154** and **156**, and dimensioning the spacing between the extensions **154** and **156**, so as to fit snugly against the tissue of each side of the septum, these are found to fit without irritation, as from a rough or uneven surface. For example, without being limiting, when using heat sealing plastic as the covering, **161**, the thickness of this material, and any finish on the adjoining edge, will affect the extent of a sensible ridge at the junction of the covering, **161**, and the molded outer shell, **169**.

The nasal septum extends in the midline from the tip of the nose anteriorly to the posterior border of the hard palate posteriorly. It is bordered inferiorly by the roof of the mouth (the hard palate) and superiorly by the floor of the cranium. As to a specific area of the nasal septum that is preferred for use of a nasal pulse oximeter probe such as the one depicted in Figures 8A,B, at least one highly vascularized, and thus more suitable, area of the nasal septum is located approximately 0.5-1.0 cm. from the posterior border of the nostril and approximately 2.0-2.5 cm. superior to the floor of the nasal cavity in the midline. Being more highly vascularized, such thereby provides more consistent and reliable signals than less vascularized areas that are, relative to this, more proximal (the tip of the nose) or more distal (further posterior towards the back of the nasal cavity). In particular, and more specifically, the highly vascularized area of the septum, known alternately as Kiesselbach's plexus and Little's area, is a preferred target area for detection of blood oxygen saturation levels by a nasal pulse oximeter probe

The pulse oximeter nasal probe is designed so that, when properly positioned, it passes its light through such highly vascularized areas. In the particular device shown in Figures 8A,B, an angle of **inflection, 170,** is shown between plastic housing, **152**, and the two extensions, **154** and **156.** This angle properly is measured as an interior deviation from a straight line extended from the plastic housing, **152**. The angle of **inflection, 170,** is between about 0 and about 33 degrees, in more preferred arrangements the angle of inflection, **170,** is between about 10 and about 27 degrees, and in even more preferred arrangements, the angle of inflection, **170**, is between about 10 and about 20 degrees. In Figure 8B, the angle of inflection, **170**, is about 15 degrees. This angle has been found to provide superior results in testing.

The two extensions, **154** and **156**, are spaced apart from one another so as to fit snugly against the tissue of each side of the septum, to avoid interference from ambient lighting. Also, it is noted that clear plastic covers, **161**, are placed over the molded plastic frame that forms the extensions **154** and **156** in Figures 8A-B. These plastic covers typically are heat-sealed over the LEDs **162** and **164** and photodetector **166**. Typically, the sides, **165**, of the clear plastic covers, **161**, that are facing or contacting the nasal septum (not shown) are aligned with the inside sides, **167**, of the extensions **154** and **156,** so as to fit snugly against the tissue of each side of the septum, without irritation, as from a rough or uneven surface. In particular, Figures 8A and 8B, show that a plastic **cover, 161,** is fitted over each of the **structures, 163,** that contain the LEDs **162** and **164,** located on extension **156,** and the photodetector **166,** located on extension **154.** The plastic covers, **161,** preferably do not interfere with light transmission. Apart from heat-shrink sealing, other means of attaching the plastic **covers, 161,** to the extensions **154** and **156,** include, but are not limited to, sonic welding, spot gluing, hot gluing, press fitting, and other such methods of attachment, as are employed in the art, that are used to attach components of a medical device for entry into an orifice of a living subject.

As depicted in Figures 8A,B, the two extensions, **154** and **156**, when fitted with the covers, **161**, are spaced apart from one another so as to fit snugly against the tissue of each side of the septum, to avoid interference from ambient lighting. The inner faces, **167**, of the two extensions, **154** and **156**, lightly rest against the tissue (mucosal nasal lining) of the septum without excessive pressure, so as to avoid the development of necrosis of the mucosal tissue.

Thus, in general, the two extensions, **154** and **156**, are angled so that upon insertion and proper placement into position in the nostrils, the LEDs **162** and **164**, located on extension **156**, emit light directed through a region that includes a preferred area of the nasal septum. Most preferably, the LEDs **162** and **164**, located on extension **156**, direct light exclusively through the highly vascularized region of the septum known alternately as Kiesselbach's plexus and Little's area. Empirically, in certain evaluations, this highly vascularized region is measured to be located approximately 2.0 cm upward and approximately 1.0 cm inward (toward the back of the head) from the tip of the anterior nasal spine. Kiesselbach's plexus is the region of the nasal septum where the terminal branches of at least two arteries meet and supply the tissue. These major terminal branches in Kiesselbach's plexus are those of the nasal septal branch of the superior labial branch of the facial artery and the anterior septal branch of the anterior ethmoidal artery (see, for example, plate 39, of Atlas of Human Anatomy, 2nd Ed., Frank H. Netter, M.D., Novartis, 1997). Some terminal branches of the posterior septal branch of the sphenopalatine artery may also be found in more posterior regions of Kiesselbach's plexus.

Also, because Kiesselbach's plexus actually is comprised of a region of highly vascularized tissue, rather than a discrete point, and given anatomical variation among persons, a range of approximately +/- 0.25 cm from the above indicated measured point also is acceptable as a target area to obtain unexpected superior results with a nasal pulse oximeter probe. It also is recognized, based on the approximate size of the Kiesselbach's plexus, that placing the probe so it measures saturation within a range as large as approximately +/- 0.75 cm from the measured point may also provide these unexpected superior results (by having the light pass through this highly vascularized region). However, given the variations noted above, this is less preferred than the range of approximately +/- 0.25 cm. from the measured point. Under certain circumstances, a range of approximately +/- 0.50 cm. from the measured point is considered acceptable. Given the basic morphology and sizing of the nares, design and placement of nasal pulse oximeter probes such that they pass light through nasal septum tissue within these larger ranges, but not within the smaller approximately +/- 0.25 cm. range, frequently requires probes that are designed to have smaller (i.e., thinner, narrower) profiles than the profile depicted in Figures 8A and 8B. This allows these probes to come closer to the outward or inward structures of the nares and maintain patient comfort.

Also, for any of these ranges to target Kiesselbach's plexus, it is appreciated that the angle of a particular individual's lip in relation to the nose, and the placement of the nasal probe sensor on the upper lip, affect the exact location of the probe's light producing and light sensing components on the plexus. As noted, it has been learned that a nasal pulse oximeter, such as is depicted by **150** (with or without the oxygen canulla element), that has an angle of inflection, **170**, of about 15 degrees, has been found to provide superior results in testing.

Also shown in Figure 8B is a conduit for oxygen (or oxygen-rich air or other gas mixture), **180**, and a conduit, **182**, within which are electrically conductive wires (or other types of signal transmission means, such as fiberoptic cable) to pass electrical signals to and from the two light-emitting diodes, **162** and **164**, and the opposing photodetector, **166**.

Means of stabilizing the probe, **150**, such as elastic straps (not shown) from any part of the device that span the head of the patient, typically are employed, and depend on the type of application and the comfort requirements of the user.

As for the probes depicted in Figures 1 and 2A-C and described above, timed electrical impulses from a pulse oximeter monitor system pass through two wires or other signal transmission means (not shown) in cables held within conduit passing within **182** to produce the light from LEDs **162** and **164**. At least one photodetector, **166**, is positioned within extension **154** to face and oppose LEDs **162** and **164** on extension **156**. The photodetector **166**, which typically is a light-sensing photodiode, detects changes in the light emitted by the LEDs **162** and **164** as that light is differentially absorbed between and during pulses across the capillaries of the septum tissue between the two extensions, **156** and **154**. In one embodiment, LED **162** emits light around 650-670 nm, and LED **164** emits light around 880-940 nm. The electrical impulses are timed to be offset from one another, so that the light from each of the two LEDs, **162** and **164**, is emitted at different times. The photodetector, **166**, detects the light passing through the septum of the nose, which is situated between extensions **156** and **154** when the probe **150** is in use. As discussed above, loss of signal through vascularized tissue such as the nasal septum is due both to background tissue absorption and the absorption by the blood in the arteries, which expands during a pulse. The signals from photodetector **166** pass through conductors (not shown) to the processor of the monitor system (not shown). As examples, not meant to be limiting, a single cable passing from one side of the device, **150**, or two cables that may form a loop that may lie above the ears of the patient, or join to form a single cable (not shown), pass signals to the two LEDs, **162** and **164,** and from the photodetector **166**. In one preferred embodiment, a single cable, formed from the joining of two cables leading from the device, **150**, terminates in an electrical plug suited for insertion into a matching socket in the pulse oximeter monitor system (not shown). In another preferred embodiment, the single cable terminates by connecting to an adapter cable, which in turn connects to a socket in the pulse oximeter monitor system (not shown). In a typical application, the signals from the light-sensing photodetector, **166**, are ultimately received and processed by a general purpose computer or special purpose computer of the monitor system (not shown).

Per the disclosure preceding this example, this combination nasal pulse oximeter is used either in combination with the needed computer processing to interpret and provide a viewable (or audible in the case of an alarm) data output of arterial blood oxygen saturation for the user or health care worker, or, in alternative arrangements this function is further combined with the means to regulate and adjust the flow of oxygen or oxygen-rich gas that is being delivered by adjustment of a valve controlling such flow.

### Example 6:

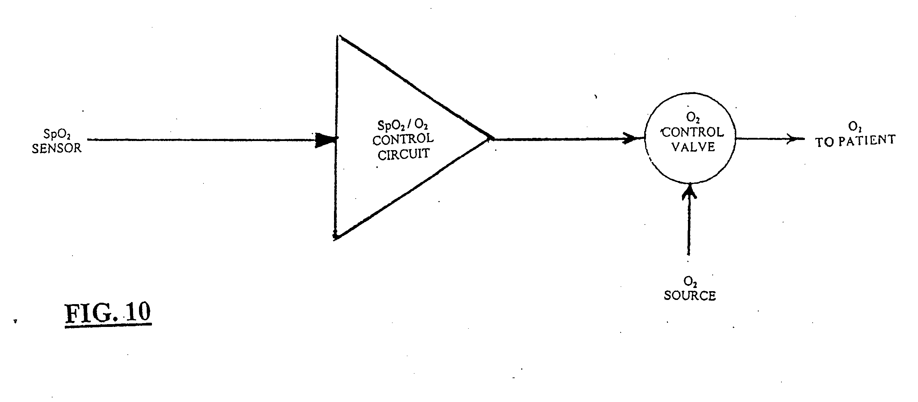

The present invention also is adapted for arrangements which utilize, in combination, pulse oximeter detection of arterial blood oxygen saturation, in combination with a supply of oxygen, air, or a gas mixture providing a variably supply of oxygen, where the flow rate and/or amount of oxygen provided in the gas mixture is controlled based at least in part by the changes and levels of arterial blood oxygen saturation, as detected by the pulse oximeter. Applications for such combination devices (pulse oximeter/oxygen supply directed by controller with pulse oximeter data as an input) include, but are not limited to: self-contained breathing apparatuses (SCBA); self-contained underwater breathing apparatuses (SCUBA); high altitude breathing systems, and medical gas delivery systems. The following figures and related disclosure provides only one, non-limiting arrangements to present the basic concepts of a SCUBA regulator. Figure 10 is used to depict a general control approach for this and other systems described in this application.

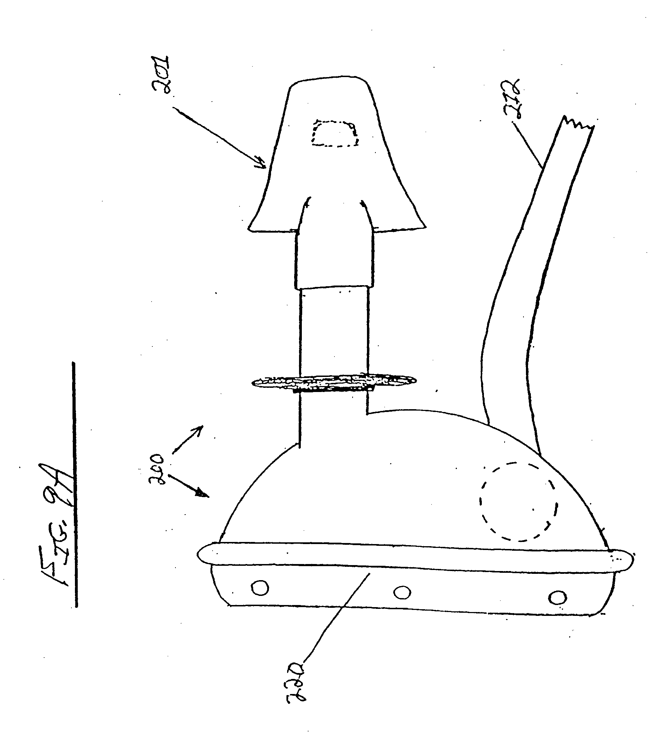

Figure 9A presents a diagram of a basic SCUBA regulator, **200**, with key features described. The housing, 220, contains a diaphragm that senses ambient water pressure which is linked physically to adjust the delivery pressure to the user via regulation of the second stage regulator, also within the housing, **220**. A supply hose, **212**, carries compressed air, or other gas mixtures, typically from a supply tank (not shown), to the second stage regulator (not shown) within the housing, **220**. Upon demand from the user, whose mouth is fitted around the rubber mouthpiece, **201**, the air or other gas from the hose, **212**, passes through the second stage regulator, and through the mouthpiece, **201**, to provide the user with a supply of air or other gas based on the flow pattern of the second stage regulator. Having the rate of this flow upon demand being variable, based in part on the type of regulator and its operating conditions and maintenance, can result in waste of precious air supply. Also, when the percent of oxygen or other gas in the air supply can be altered based on data from a pulse oximeter, the body's physiological requirements can be better met, resulting in a safer and healthier dive experience.

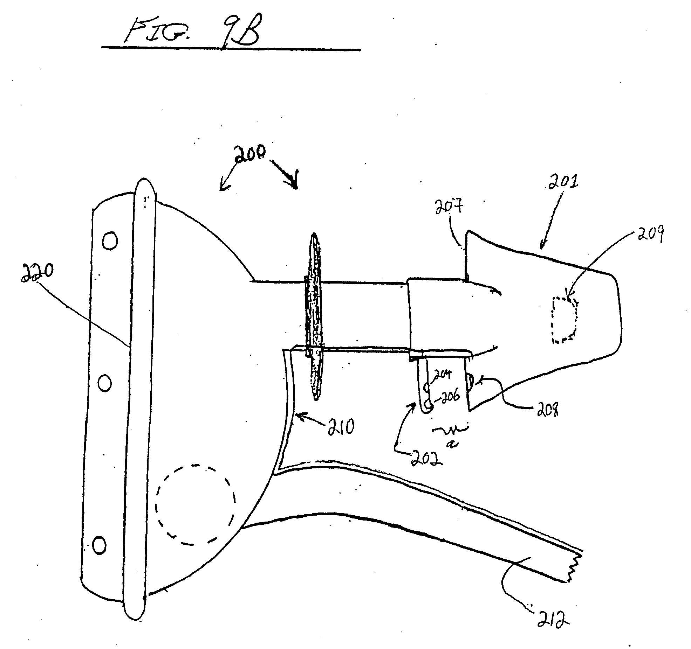

Figure 9B presents a diagram of the basic SCUBA regulator Figure 9A, however also comprising additional features In particular, a flexible arm **202** bearing two light-emitting sources, **204** and **206**, (typically LED's) is disposed in a place exterior to the position of the lip of the diver using the regulator. The approximate thickness of the lip is represented in Figure 9B by the distance, a. Flexibility of the arm **202**, is by the nature of the material such as rubber and/or by spring loading. By such design, the arm **202** moves easily away during fitting of the mouthpiece (i.e., the placement of the lip around the flange, **207**, of the mouthpiece, and the teeth over the nubs, **209** (one is shown as a dashed rectangle to indicate position on the inside surface of the flange). Then the arm, **202**, presses against the lip or against the skin just below the lower lip, in such an orientation so that light emitted by the two light-emitting sources, **204** and **206**, is directed toward a photosensor, **208**, inset into the outer surface of the rubber mouthpiece **201**. This receives signals through the lower lip/flesh below the lip, which is sufficiently well-vascularized to provide a representative reading of the body's oxygen status expressed as of arterial blood oxygen saturation. Wiring **210** passes data signals between this pulse oximeter probe and the oximeter controller (not shown, end of wiring in figure coincides with end of supply hose, **212**). This interprets the data signals from the pulse oximeter probe and, based on the information received, directs a separate valve (not shown) to adjust upward or downward the level and/or pressure of oxygen supplied through the supply hose **212** to increase or decrease the absolute or relative oxygen flowing to the regulator at the mouthpiece shown in Figure 9B.

For SCUBA systems using HE/Ox mixtures, additional oxygen can be provided when indicated by the data from the oximeter, and/or by a manual control (such as the diver pressing a button to increase, with a second button to decrease oxygen flow incrementally). The same approaches apply to more complex dive gas mixtures, such as Triox (oxygen enriched air with helium) and Trimix (hypoxic oxygen, helium and nitrogen). By appropriate control mechanisms and algorithms (adjusted to compensate for physiological differences at different depths), a diver using such arrangements of the present invention extends the dive time on a particular quantity of oxygen, and/or has more oxygen when more oxygen is needed, and less oxygen when less oxygen is sufficient. This results in a safer, healthier dive experience.

The above disclosed improvements in the monitoring of blood oxygen saturation and adjustment of gases supplied to a SCUBA diver also apply to users of self-contained breathing apparatuses (SCBA) that are not used for underwater diving. For example, firemen and other emergency workers use SCBA in environments in which they may exert considerable energy and have transient very high oxygen demands. The above-disclosed arrangements and variations of these known to those of skill in the relevant arts, provide benefits to such users.

Figure 10 provides a flow diagram of the general information and control generation of the "pulse flow oxygen sensor" that controls the level or pressure of oxygen provided to a user wearing a nasal or mouth pulse oximeter sensor in combination with the gas supply controlled by a controller receiving data input from that pulse oximeter sensor. Fundamentally, data signals from the pulse oximeter sensor go to an arterial blood oxygen saturation/oxygen supply Control Circuit. Based on analysis of these data signals using an appropriate algorithm, the arterial blood oxygen saturation/oxygen supply Control Circuit sends signals to a servodevice that adjusts an Oxygen Control Valve which receives oxygen under pressure from an oxygen source. From the O₂ Control Valve, oxygen is directed to a patient in need of oxygen, where that patient is wearing the combined nasal or mouth pulse oximeter sensor in combination with the gas supply cannula or mouthpiece.

Preferably, the oximeter probes and sleeves are easily fabricated from low cost materials and are adaptable for use in an operating room, intensive care unit, emergency department, post-surgery recovery areas and other areas to treat patients in need of hemodynamic monitoring. The monitoring system is particularly applicable for use with patients in whom hypotension or poor perfusion are problematic. In addition, the monitoring system is particularly well suited for use with multi-trauma and thermally injured patients who either have severe peripheral vasoconstriction or have severely damaged or destroyed peripheral vascular beds. Through combining at least two pulse oximeters capable of measuring desired parameters at at least two locations into a single monitor system, the present invention provides a more accurate assessment of perfusion and resistance in patients, than any of the presently available single probe pulse oximeters.

It should be understood that the examples and embodiments described herein are for illustrative purposes only and that various modifications or changes in light of the scope of the appended claims are allowed

## Claims

1. A pulse oximetry probe (10) for measurement of pulse-based differences in light absorbence across the vascularized tissue of a lip or a cheek of a patient, comprising:
a. a resilient probe frame having an inner face and an outer face, said frame comprising a proximal arm (1) connecting at one end to a cable (7) and at the other end to a bridging section (2) of said frame, said bridging section connecting the proximal arm (1) to one end of a distal arm (3) of said frame, wherein a portion of said distal arm (3) is at a determined distance from an opposing portion of said proximal arm (1);
b. a first pad (12) positioned over at least two light-generating structures (17) that emit light at least two different wavelength bands known to differentiate oxygenated from non-oxygenated hemoglobin, on the inner face of said portion of the distal arm (3) or of the proximal arm (1);
c. a second pad (14) positioned over at least one light-detecting structure (15) that detects light transmitted from said first pad (12), on the inner face of the arm opposing the first pad (12); and
d. first individual conductors for energizing said at least two light-generating structures (12), connecting said structures to a monitoring system for light signal production and modulation, and second individual conductors connecting said at least one light-detecting structure (15) to said monitoring system to convey signals of light detected by said at least one light-detecting structure (15), said first and said second individual conductors passing within said resilient probe frame and thereafter through a cable for carrying said first and said second individual conductors to said monitoring system,
**characterised in that** said probe frame is hook-shaped, and is sized to engage both sides of the lip or cheek tissue of an average pediatric patient or of an average adult patient between said first (12) and second (14) pads,
and **in that** said hook-shaped probe frame is of such resiliency that the force required to move the distal end outwardly 0.00635 m (0. 25 inch) is between about 1.4709 to 12.2575 N (150 to 1,250 grams of force) and the force required to move the distal end inwardly 0.00635 m (0.25 inch) is between about 1.9613 to 21.5746 N (200 to 2,200 grams of force).

2. The probe (10) of claim 1, additionally comprising a boot (8) to seal the junction between said probe frame and said cable (7).

3. The probe (10) of claim 2, wherein said first (12) and second (14) pads are dome-shaped and spaced apart sufficiently to comfortably engage both sides of the lip or cheek tissue of a patient.

4. The probe (10) of claim 2, wherein said probe (10) is construed of materials that impart a resiliency allowing the angular and dimensional relationships between the light-generating structures (17) and the opposingly placed light-detecting structure (15) to remain substantially consistent, which materials have sufficient memory to substantially return to their original shape after a normal flexion.

5. The probe (10) of claim 1, additionally comprising a disposable covering sleeve configured to slidably fit over said probe (10), wherein said covering sleeve is comprised of thin flexible material of a thickness in a general range of 0.000127 to 0.00127 m (0.005 to 0.050 inches), which sleeve, at least in the areas of the light-generating structures (17) and light-detecting structure (15), is highly transparent to the wavelengths used by the probe (10) itself.

## Patentansprüche

1. Pulsoximetriesonde (10) zum Messen von pulsbasierten Differenzen in der Lichtabsorption an dem vaskularisierten Gewebe einer Lippe oder einer Wange eines Patienten, mit:
a. einem elastischem Sondenrahmen, der eine innere Fläche und eine äußere Fläche hat, wobei der Rahmen einen proximalen Arm (1) aufweist, der an einem Ende mit einem Kabel (7) und an dem anderen Ende mit einem Überbrückungsabschnitt (2) des Rahmens verbunden ist, wobei der Überbrückungsabschnitt den proximalen Arm (1) mit einem Ende eines distalen Arms (3) des Rahmens verbindet und wobei ein Teil des distalen Arms (3) in einer bestimmten Distanz von einem gegenüberliegenden Teil des proximalen Arms (1) ist;
b. einem ersten Polster (12), das über wenigstens zwei Licht erzeugenden Gebilden (17), die Licht in wenigstens zwei verschiedenen Wellenlängebändern emittieren, welche dafür bekannt sind, mit Sauerstoff angereichertes Hämoglobin von nicht mit Sauerstoff angereichertem Hämoglobin zu differenzieren, auf der inneren Fläche des Teils des distalen Arms (3) oder des proximalen Arms (1) angeordnet ist.
c. einem zweiten Polster (14), das über wenigstens einem Licht erfassenden Gebilde (15) angeordnet ist, welches Licht, das aus dem ersten Polster (12) übertragen worden ist, auf der inneren Fläche des Arms, welche dem ersten Polster (12) gegenüberliegt, erfasst;
d. ersten einzelnen Leitern zur Stromversorgung der wenigstens zwei Licht erzeugenden Gebilde (12), welche die Gebilde mit einem Überwachungssystem für Lichtsignalproduktion und -modulation verbinden, und zweiten einzelnen Leitern, welche das wenigstens eine Licht erfassende Gebilde (15) mit dem Überwachungssystem verbinden, um Signale von Licht, welches durch das wenigstens eine Licht erfassende Gebilde (15) erfasst wird, zu transportieren, wobei die ersten und die zweiten einzelnen Leiter durch das Innere des elastischen Sondenrahmens und anschließend durch ein Kabel hindurchgeführt sind zum Führen der ersten und der zweiten einzelnen Leiter zu dem Überwachungssystem,
**dadurch gekennzeichnet, dass** der Sondenrahmen hakenförmig und dafür dimensioniert ist, beide Seiten des Lippen- oder Wangengewebes eines durchschnittlichen pädiatrischen Patienten oder eines durchschnittlichen erwachsenen Patienten zwischen dem ersten (12) und dem zweiten (14) Polster zu erfassen, und
dass der hakenförmige Sondenrahmen eine derartige Elastizität hat, dass die Kraft, die erforderlich ist, um das distale Ende 0,00635 m (0.25 Zoll) nach außen zu bewegen, zwischen etwa 1,4709 und 12,2575 N (150 bis 1.250 Gramm Kraft) liegt, und dass die Kraft, die erforderlich ist, um das distale Ende um 0,00635 m (0.25 Zoll) einwärts zu bewegen, zwischen etwa 1,9613 und 21,5746 N (200 bis 2.200 Gramm Kraft) liegt.

2. Sonde (10) nach Anspruch 1, zusätzlich mit einer Manschette (8) zum Abdichten der Verbindung zwischen dem Sondenrahmen und dem Kabel (7).

3. Sonde (10) nach Anspruch 2, wobei das erste (12) und das zweite (14) Polster kuppelförmig und ausreichend voneinander beabstandet sind, um beide Seiten des Lippen- oder Wangengewebes eines Patienten bequem zu erfassen.

4. Sonde (10) nach Anspruch 2, wobei die Sonde (10) aus Materialien aufgebaut ist, welche eine Elastizität verleihen, die den Winkel- und Dimensionsbeziehungen zwischen den Licht erzeugenden Gebilden (17) und dem gegenüber platzierten Licht erfassenden Gebilde (15) erlauben, im Wesentlichen konsistent zu bleiben, wobei diese Materialien ausreichend Formerinnerungsvermögen haben, um nach einer normalen Biegung zu ihrer ursprünglichen Form zurückzukehren.

5. Sonde (10) nach Anspruch 1, zusätzlich mit einer Einweg-Abdeckhaube, die dafür ausgebildet ist, über die Sonde (10) geschoben werden zu können, wobei die Abdeckhaube aus einem dünnen Material mit einer Dicke in einem allgemeinen Bereich von 0,000127 bis 0,00127 m (0.005 bis 0.050 Zoll) besteht und wobei die Haube wenigstens in den Bereichen der Licht erzeugenden Gebilde (17) und des Licht erfassenden Gebildes (15) für die Wellenlängen, die durch die Sonde (10) selbst benutzt werden, äußerst transparent ist.

## Revendications

1. Sonde d'oxymétrie de pouls (10) pour mesurer des différences basées sur le pouls basé dans l'absorption de lumière à travers le tissu vascularisé d'une lèvre ou d'une joue d'un patient, comprenant :
a. un cadre de sonde élastique ayant une face interne et une face externe, ledit cadre comprenant un bras proximal (1) relié à une extrémité à un câble (7) et à l'autre extrémité à une section de pont (2) dudit cadre, ladite section de pont reliant le bras proximal (1) à une extrémité d'un bras distal (3) dudit cadre, dans laquelle une partie dudit bras distal (3) est à une distance déterminée d'une partie opposée dudit bras proximal (1) ;
b. une première pastille (12) positionnée sur au moins deux structures de génération de lumière (17) qui émettent une lumière dans au moins deux bandes de longueur d'onde différentes connues pour différentier l'hémoglobine oxygénée de l'hémoglobine non oxygénée, sur la face interne de ladite partie du bras distal (3) ou du bras proximal (1) ;
c. une deuxième pastille (14) positionnée sur au moins une structure de détection de lumière (15) qui détecte la lumière transmise à partir de ladite première pastille (12), sur la face interne du bras faisant face à la première pastille (12) ; et
d. des premiers conducteurs individuels pour alimenter lesdites au moins deux structures de génération de lumière (12), connectant lesdites structures à un système de surveillance pour la production et la modulation de signaux de lumière, et des deuxièmes conducteurs individuels connectant ladite au moins une structure de détection de lumière (15) au dit système de surveillance pour transporter les signaux de lumière détectés par ladite au moins une structure de détection de lumière (15), lesdits premiers et lesdits deuxièmes conducteurs individuels passant dans ledit cadre de sonde élastique et ensuite à travers un câble pour acheminer lesdits premiers et lesdits deuxièmes conducteurs individuels vers ledit système de surveillance,
**caractérisée en ce que** ledit cadre de sonde est en forme de crochet, et est dimensionné pour venir en prise avec les deux côtés du tissu de la lèvre ou de la joue d'un patient pédiatrique moyen ou d'un patient adulte moyen entre lesdites première (12) et deuxième (14) pastilles,
et **en ce que** ledit cadre de sonde en forme de crochet a une élasticité telle que la force nécessaire pour déplacer l'extrémité distale vers l'extérieur de 0,00635 m (0,25 pouce) est entre environ 1,4709 et 12,2575 N (150 et 1.250 grammes de force) et la force nécessaire pour déplacer l'extrémité distale vers l'intérieur de 0,00635 m (0,25 pouce) est entre environ 1,9613 et 21,5746 N (200 et 2.200 grammes de force).

2. Sonde (10) selon la revendication 1, comprenant en plus une gaine (8) pour sceller la jonction entre ledit cadre de sonde et ledit câble (7).

3. Sonde (10) selon la revendication 2, dans laquelle lesdites première (12) et deuxième (14) pastilles sont en forme de dôme et suffisamment espacées pour venir en prise confortablement avec les deux côtés du tissu de la lèvre ou de la joue d'un patient.

4. Sonde (10) selon la revendication 2, dans laquelle ladite sonde (10) est réalisée en des matériaux qui communiquent une élasticité permettant que les relations angulaires et dimensionnelles entre les structures de génération de lumière (17) et la structure de détection de lumière (15) placée de manière opposée restent sensiblement constantes, lesquels matériaux ont une mémoire suffisante pour retourner sensiblement à leur forme d'origine après une flexion normale.

5. Sonde (10) selon la revendication 1, comprenant en plus une gaine de recouvrement jetable configurée pour s'ajuster de manière coulissante sur ladite sonde (10), dans laquelle ladite gaine de recouvrement est composée d'un mince matériau flexible d'une épaisseur dans une plage générale de 0,000127 à 0,00127 m (0,005 à 0,050 pouce), laquelle gaine, au moins dans les zones des structures de génération de lumière (17) et de la structure de détection de lumière (15), est hautement transparente aux longueurs d'onde utilisées par la sonde (10) elle-même.
